# EUROPEAN PATENT SPECIFICATION

(11) **EP 1 663 188 B1**
(45) Date of publication and mention of the grant of the patent: **10.08.2016**
(21) Application number: 04787557.0
(22) Date of filing: 10.09.2004
(51) Int. Cl.: A61K 31/137, A61P 25/00, A61P 25/16, A61P 25/18, A61P 25/26, A61P 25/28

(54) **TREATMENT OF DISORDERS OF THE NERVOUS SYSTEM**
BEHANDLUNGE VON ERKRANKUNGEN DES NERVENSYSTEMS
TRAITEMENT DE MALADIES OU DE LESIONS DU SYSTEME NERVEUX AVEC LE FTY720

(30) Priority: 12.09.2003 US 502386 P
(43) Date of publication of application: 07.06.2006
(73) Proprietor: Newron Sweden AB, 114 33 Stockholm (SE)
(72) Inventor: LINDQUIST, Per, S-175 63 Jarfalla (SE)
(74) Representative: Cooley (UK) LLP
(86) International application number: PCT/IB2004/003288
(87) International publication number: WO 2005/025553

(56) References cited:
- WO-A-2004/028521
- FUJINO M ET AL: "AMELIORATION OF EXPERIMENTAL AUTOIMMUNE ENCEPHALOMYELITIS IN LEWIS RATS BY FTY720 TREATMENT" JOURNAL OF PHARMACOLOGY AND EXPERIMENTAL THERAPEUTICS, AMERICAN SOCIETY FOR PHARMACOLOGY AND, US, vol. 305, no. 1, April 2003 (2003-04), pages 70-77, XP009022804 ISSN: 0022-3565
- KANEIDER N C ET AL: "The immune modulator FTY720 targets sphingosine-kinase-dependent migration of human monocytes in response to amyloid beta-protein and its precursor" FASEB JOURNAL 2004 UNITED STATES, vol. 18, no. 11, 2004, pages 1309-1311, XP008043407 ISSN: 0892-6638
- KIUCHI M ET AL: "Synthesis and immunosuppressive activity of 2-substituted 2-aminopropane-1,3-diols and 2-aminoethanols." JOURNAL OF MEDICINAL CHEMISTRY. 27 JUL 2000, vol. 43, no. 15, 27 July 2000 (2000-07-27), pages 2946-2961, XP002319479 ISSN: 0022-2623

## Description

This application claims the benefit of U. S. Patent Application 60/502, 386 filed September 12, 2003.

### FIELD OF THE INVENTION

The invention relates generally to methods of influencing neural stem cells and neural progenitor cells (collectively termed NSC) to produce progeny that can replace damaged, missing, or dying neurons or other nervous system cell types. More specifically, the invention includes use of FTY720 or a derivative thereof selected from R-AAL, phosphate ester metabolites of FTY720, pharmaceutically acceptable salts of FTY720 or pegylated FTY720 in the manufacture of a composition for the treatment of a neurodegenerative disease, a neurological disease or a psychiatric disease.

### BACKGROUND OF THE INVENTION

For several years, it has been established that neural stem cells exist in the adult mammalian brain. The first suggestions that new neurons were generated in the adult mammalian brain came from studies performed in the 1960s (Altman and Das 1965; Altman and Das 1967). However, it took another three decades and refined technical procedures, to overthrow the theory that neurogenesis within the mammalian, central nervous system (CNS) was restricted to embryogenesis and the perinatal period (for review see Momma, Johansson et al. 2000; Kuhn and Svendsen 1999). Treatment of neural disease and injury traditionally focused on keeping existing neurons alive, but possibilities now exist for exploiting neurogenesis for therapeutic treatments of neurological disorders and diseases.

The source of new neurons is adult neural stem cells (NSC), located, e. g., within the ependymal and/or subventricular zone (SVZ) lining the lateral ventricle (Doetsch, Caille et al. 1999; Johansson, Momma et al. 1999) and in the dentate gyrus of hippocampus formation (Gage, Kempermann et al. 1998). Recent studies reveal the potential for several additional locations of NSC within the adult CNS (Palmer, Markakis et al. 1999). Asymmetric division of NSC maintain their number while generating a population of rapidly dividing precursor, or progenitor cells (Johansson, Momma et al. 1999). The progenitors respond to a range of cues that dictate the extent of their proliferation and their fate, both in terms of cell type they differentiate into and the position they ultimately take up in the brain.

The NSC of the ventricular system in the adult are likely counterparts of the embryonic ventricular zone stem cells lining the neural tube whose progeny migrate away to form the CNS as differentiated neurons and glia (Jacobson 1991). NSC persist in the adult lateral ventricle wall (LVW), generating neuronal progenitors that migrate down the rostral migratory stream to the olfactory bulb, where they differentiate into granule cells and periglomerular neurons (Lois and Alvarez-Buylla 1993). Substantial neuronal death occurs in the olfactory bulb generating a need for continuous replacement of lost neurons, a need satisfied by the migrating progenitors derived from the LVW (Biebl, Cooper et al. 2000). Further to this ongoing repopulation of olfactory bulb neurons, there are strong indications that lost neurons from other brain regions can be replaced by progenitors from the LVW that differentiate into the lost neuron phenotype complete with appropriate neuronal projections and synapses with the correct target cell type (Snyder, Yoon et al. 1997; Magavi, Leavitt et al. 2000).

*In vitro* cultivation techniques have been established to identify the external signals involved in the regulation of NSC proliferation and differentiation (Johansson, Momma et al. 1999; Johansson, Svensson et al. 1999). The mitogens EGF and basic FGF allow neural progenitors, isolated from the ventricle wall and hippocampus, to be greatly expanded in culture (McKay 1997; Johansson, Svensson et al. 1999). The dividing progenitors remain in an undifferentiated state growing into large balls of cells known as neurospheres. Withdrawal of the mitogens combined with addition of serum induces differentiation of the progenitors into the three cell lineages of the brain, neurons, astrocytes, and oligodendrocytes (Doetsch, Caille et al. 1999; Johansson, Momma et al. 1999). Application of specific growth factors can distort the proportions of each cell type in one way or the other. For example, CNTF acts to direct the neural progenitors to an astrocytic fate (Johe, Hazel et al. 1996; Rajan and McKay 1998), while the thyroid hormone, triiodothyronine (T3) has been shown to promote oligodendrocyte differentiation (Johe, Hazel et al. 1996). Enhancement of neuronal differentiation of neural progenitors by PDGF has also been documented (Johe, Hazel et al. 1996; Williams, Park et al. 1997).

The ability to expand neural progenitor and then manipulate their cell fate has enormous implications in transplant therapies for neurological diseases in which specific cell types are lost, the most obvious example being Parkinson's disease (PD) characterized by degeneration of dopaminergic neurons in the substantia nigra. Previous transplantation treatments for PD patients have used fetal tissue taken from the ventral midbrain at a time when substantia nigral dopaminergic neurons are undergoing terminal differentiation (Herman and Abrous 1994). The cells are grafted onto the striatum where they form synaptic contacts with host striatal neurons, their normal synaptic target, restoring dopamine turnover and release to normal levels with significant functional benefits to the patient (Herman and Abrous 1994) (for review, see Bjorklund and Lindvall 2000). Grafting of fetal tissue is hindered by lack of donor tissue, and its use raises moral questions. *In vitro* expansion and manipulation of NSC, however, can potentially provide a range of well characterized cells for transplant-based strategies for neurodegenerative disease, such as dopaminergic cells for PD. The use of adult-derived stem cells for tissue repair may help to overcome the ethical problems associated with embryonic cell research. To this aim, the identification of factors and pathways that govern the proliferation and differentiation of neural cell types will prove fundamental.

Ultimately the identification of these proliferative and differentiating factors is likely to provide insights into the stimulation of endogenous neurogenesis for the treatment of neurological diseases and disorders. Intraventricular infusion of both EGF and basic FGF has been shown to proliferate the ventricle wall cell population. In the case of EGF, extensive migration of progenitors into the neighboring striatal parenchyma has been demonstrated (Craig, Tropepe et al. 1996; Kuhn, Winkler et al. 1997). Differentiation of the progenitors was predominantly into a glial lineage, and the generation of neurons was reduced (Kuhn, Winkler et al. 1997). A recent study found that intraventricular infusion of BDNF in adult rats promotes an increase in the number of newly generated neurons in the olfactory bulb and rostral migratory stream, and in parenchymal structures, including the striatum, septum, thalamus and hypothalamus (Pencea, Bingaman et al. 2001). These studies demonstrate that the proliferation of progenitors within the SVZ of the LVW can be stimulated and that their lineage can be manipulated to produce neuronal and glial fates.

Currently, the number of factors know to affect neurogenesis *in vivo* is small and their effects are either undesired or limited. There is a need to further extend the search for factors that can selectively stimulate neural stem cell activity, proliferation of neural progenitors, and differentiation of progenitors into the desired neuronal cell types. Needed are new methods for *stimulating in vivo* neurogenesis and culturing cells for transplantation therapy.

FTY720 (2-amino-2-[2-(4-octylphenyl)ethyl]-1,3-propanediol) has been identified as an orally active immunosuppressant (see, e.g., WO 94/08943; WO 99/36065) obtained by chemical modification of myriocin (Adachi K et al. 1995). Myriocin, a natural product related to sphingosine, was first described as an antifungal antibiotic in 1972. More than 20 years later, myriocin was rediscovered as an immunosuppressive metabolite (ISP-I) from the ascomycete, *Isaria sinclairii.* As an active immunosuppressant, FTY720 is currently being developed for treatment of multiple sclerosis (MS; Novartis Phase II trials published by the Investigational Drugs Database (IDDB) available at world wide web.iddb.org/). Recent studies have suggested that FTY720 can alleviate multiple sclerosis symptoms, based on the widely used animal model for MS known as experimental autoimmune encephalomyelitis (EAE).

Brinkmann et al. (2002) treated Wistar rats orally for two weeks with FTY720 (0.3 mg/kg/day) from the day of induction of EAE. This completely prevented the onset of MS-like symptoms. Using myelin basic protein as the immunogen in Lewis rats, Fujino et al. (2003) also showed an almost complete suppression of EAE-development by FTY720 (orally 1mg/kg/day). This was associated with a dramatic reduction of leukocyte infiltration into the CNS as well as in a decreased expression of IL-2, IL-6, and INF-γ in the CNS. Finally, using SJL-mice (a strain susceptible to induction of EAE), Webb et al. (2004) induced relapsing-remitting EAE, which is thought to closely mimic human MS. The authors showed that FTY720 treatment resulted in a rapid and sustained improvement of the clinical status of the animals and a reversal of changes in expression of mRNAs encoding some myelin and inflammatory proteins.

Thus, FTY720 has been well established as an active immunosuppressant. Experiments using pertussis toxin, an inhibitor of G protein signaling, have indicated that FTY720 may alter lymphocyte recirculation by modulating the function of G-protein coupled receptors (GPCRs) on lymphocytes. In addition, FTY720 has been identified as an SIP receptor agonist that shows a distinct affinity pattern for SIP receptors (SIPRs ; Mandala et al, 2002). The SIPRs have been implicated in regulation of a number of physiological processes, such as vascular cell systems, vascular permeability, cardiac cell systems, and lymphocyte trafficking (Fukushima, N. , Ishii, l., 2001; Goetzl, E. J. & An, S., 1998 ; Chun, J., 1999).

### SUMMARY OF THE INVENTION

The invention is based on the surprising finding shown herein that FTY720 (2-amino-2-[2-(4-octylphenyl)ethyl]-1,3-propanediol) can affectively modulate neurogenesis of adult neural stem cells or neural progenitor cells (NSC). Thus, FTY720 is useful for modulating the proliferation, differentiation, migration, or survival of NSC. As used herein, the term "FTY720" includes FTY720 and its derivatives as described in detail herein.

In one embodiment, the invention encompasses use of FTY720 or a derivative thereof selected from R-AAL, phosphate ester metabolites of FTY720, pharmaceutically acceptable salts of FTY720 or pegylated FTY720 in the manufacture of a medicament for treating neurodegenerative disease, neurological disease, or psychiatric disease, with the proviso that the disease is not MS or demyelinating disease such as Guillain-Barre syndrome.

Also disclosed herein is FTY720 for use in alleviating a symptom of a nervous system disorder in a subject comprising administering a composition comprising FTY720 to the subject in an amount sufficient to alleviate the symptom.

Furthermore, it is disclosed that FTY720 can be used to increase the activity (i.e., growth, proliferation, differentiation, migration, or survival) of NSC in vitro or in vivo.

In particular, FTY720 can be used to create, maintain, grow, and expand neurosphere cultures with or without other growth factors.

In addition, FTY720 can be formulated into a composition, e. g., pharmaceutical composition or laboratory composition, for use with the invention.

Disclosed herein is use of FTY720 for use in modulating mammalian adult NSC activity comprising the step of contacting a cell population comprising mammalian NSC (e.g., adult or other non-embryonic cells) with a composition comprising FTY720, wherein treated cells show improved proliferation or neurogenesis compared to untreated cells.

Also disclosed is FTY720 for use in stimulating primary mammalian NSC (e.g., adult or other non-embryonic cells) to proliferate to form neurospheres comprising contacting one or more NSC with a composition comprising FTY720, wherein the contacted cells show increased proliferation of NSC and formation of neurospheres.

Additionally disclosed is FTY720 for use in a method for producing a cell population enriched for human NSC, comprising: (a) contacting a cell population that includes NSC with a composition comprising FTY720; (b) isolating the contacted cell population of step (a), thereby producing a cell population enriched for NSC.

This application also discloses FTY720 for use in increasing the proliferation of non-embryonic neural stem *cells in vitro* comprising contacting the cells with a composition comprising FTY720 in an amount sufficient to increase the proliferation of the cells.

A cell culture comprising a cell population enriched for human NSC produced by the method above is also disclosed.

A method for increasing proliferation of non-embryonic neural stem cells comprising contacting the cells with a composition comprising FTY720 in an amount sufficient to increase the proliferation of the cells is disclosed herein.

We also disclose FTY 720 for use in increasing *the in situ* activity of NSC located in the neural tissue of a mammal, comprising administering a therapeutically effective amount of FTY720, wherein the administration increases the growth, proliferation, differentiation, migration, or survival of the cells in the neural tissue.

Uses of a composition comprising FTY720 to a subject in increasing proliferation of other stem cell populations, such as hematopoietic, pancreatic, skin, and gut stem cells, are also disclosed.

We also disclose a composition comprising FTY720 for use in increasing neurogenesis in a subject suffering from a nervous system disorder comprising the step of administering (e.g., via systemic or local routes) a composition comprising FTY720 into the subject in an amount sufficient to increase neurogenesis.

Also disclosed is a cell population enriched for human NSC for use in alleviating a symptom of a nervous system disorder in a subject comprising administering a cell population enriched for human NSC (produced by the method, above) in an amount sufficient to alleviate the symptom.

We also disclose a composition comprising (a) a population of isolated NSCs obtained from adult or other non-embryonic tissue; and (b) FTY720 with or without added growth factors; in an amount sufficient to alleviate the symptom for use in alleviating a system of a nervous system disorder.

A composition comprising FTY720 for use in increasing cognitive ability is also disclosed.

In particular aspects, the FTY720 compositions and other compositions (e.g., enriched cell populations) of the invention are administered into the spinal cord of the subject, for example, by injection, infusion, or other means.

In additional aspects, FTY720 can be used alone or in combination with one or more growth factors, such as, for example, EGF, PDGF, TGF-alpha, FGF-1, FGF-2, NGF, PACAP, and others, or with one or more anti-depressants, anti-anxiety treatments, anti-psychotic treatments, epilepsy treatments, Alzheimer's treatments, Parkinson's treatments, dopamine receptor agonists, tranquilizers, sedatives, lithium, or other therapeutics

In specific aspects, the FTY720 compound may be administered in an amount of 0.1 ng/kg/day to 1 mg/kg/day, 1 ng/kg/day to 1 µg/kg/day, 1 mg/kg/day to 10 mg/kg/day, or 10 mg/kg/day to 100 mg/kg/day. Preferably, FTY720 is administered at doses of 0.3 mg to 10 mg. More preferably, FTY720 is administered to a subject in an amount of 1 ng/kg/day to 1 mg/kg/day or 1 µg/kg/day to 0.1 mg/kg/day. In certain aspects, the FTY720 composition may be administered in an amount of to achieve a target tissue concentration of 0.0001 nM to 0.1 nM, 0.001 nM to 10 nM, 0.1 nM to 1 nM, 1 nM to 10 nM, 10 nM to 100 nM, or 1µM to 10µM. Preferably FTY720 is administered to obtain a target tissue concentration of 0.001 nM to 0.05 nM or 0.02 nM to 0.04 nM. Other embodiments, objects, aspects, features, and advantages of the invention will be apparent from the accompanying description and claims.

### BRIEF DESCRIPTION OF THE DRAWINGS

FIGS. 1A-1B: FTY720 proliferates mouse NSCs *in vitro* grown as suspension cultures or as adherent cells. FIG. 1A shows results for the ATP assay. FIG. 1B shows results for BrdU incorporation.
FIG. 2: Effect of FTY720 concentrations on *in vitro* proliferation of NSCs as measured by ATP assay. EC₅₀ value calculated at 0.02 nM (maximum at 0.04 nM).
FIG. 3: Sagittal sections of adult mouse brain. FIG. 3A: S1P1 is expressed in the SVZ of the LVW expanding into the rostral migratory stream. FIG. 3B: S1P₅ is expressed in the dentate gyrus and CA1-CA3 of the hippocampus as well as in the choroid plexus.

### DETAILED DESCRIPTION OF THE INVENTION

### Definitions

Throughout this disclosure, the term "neural stem cells" (NSCs) includes "neural progenitor cell," "neuronal progenitor cell," "neural precursor cell," and "neuronal precursor cell" (all referred to herein as NPCs). These cells are non-embryonic (e.g., adult) cells that can be identified by their ability to undergo continuous cellular proliferation, to regenerate exact copies of themselves (self-renew), to generate a large number of regional cellular progeny, and to elaborate new cells in response to injury or disease.

The term "NPCs" means cells that can generate progeny that are either neuronal cells (such as neuronal precursors or mature neurons) or glial cells (such as glial precursors, mature astrocytes, or mature oligodendrocytes). Typically, the cells express some of the phenotypic markers that are characteristic of the neural lineage. They also do not usually produce progeny of other embryonic germ layers when cultured by themselves *in vitro* unless dedifferentiated or reprogrammed in some fashion.

The cell population comprising NSC may be obtained from neural tissue, e.g., human tissue, such as, for example, from non-embryonic (e.g., fetal, adult) brain, neural cell culture, or a neurosphere. For example, the NSC can be derived from tissue enclosed by dura mater, peripheral nerves, or ganglia. The NSC may be derived from lateral ventricle wall of a mammalian brain. The NSCs may alternatively be derived from stem cells originating from a tissue such as pancreas, skin, muscle, adult bone marrow, liver, and umbilical cord tissue or umbilical cord blood. The NSC, after the application of the method, will show an improved characteristic such as survival, proliferation, or migration compared to untreated cells.

As used herein, the term "neurosphere" refers to the ball of cells consisting of NSCs. The phrase "NSC activity," "activity of NSC," and similar phraseology means the growth, proliferation, differentiation, migration, or survival of NSC.

The term "treating" in its various grammatical forms in relation to the present invention refers to preventing, curing, reversing, attenuating, alleviating, ameliorating minimizing, suppressing, or halting the deleterious effects of a neurological disorder, disorder progression, disorder causative agent (e.g., cellular defect, drug, toxin, bacteria or viruses), injury, trauma, or other abnormal condition. Symptoms of neurological disorders include, but are not limited to, tension, abnormal movements, abnormal behavior, tics, hyperactivity, combativeness, hostility, negativism, memory defects, sensory defects, cognitive defects, hallucinations, acute delusions, poor self-care, and sometimes withdrawal and seclusion. Because some of the inventive methods involve counteraction against the etiological agent, the artisan will recognize that they are equally effective in situations where the inventive compound is administered prior to, or simultaneous with, action of the etiological agent (prophylactic treatment) and situations where the inventive compounds are administered after (even well after) action of the etiological agent.

According to the specific case, the "therapeutically effective amount" of an agent should be determined as being the amount sufficient to improve the symptoms of the patient in need of treatment or at least to partially arrest the disease and its complications. Amounts effective for such use will depend on the severity of the disease and the general state of the patient's health. Single or multiple administrations may be required depending on the dosage and frequency as required and tolerated by the patient. In this disclosure, a "disorder" shall have the same meaning as a "disease."

In this invention, the "target" tissue includes, but is not limited to, the ventricular wall, the volume adjacent to the wall of the ventricular system, piriform cortex, the hippocampal formation including alveus, striatum, substantia nigra, retina, amygdala, nucleus basalis of Meynert, spinal cord, thalamus, hypothalamus, septum and cerebral cortex.

The term "injection", throughout this application, encompasses all forms of injection known in the art and at least the more commonly described injection methods such as subcutaneous; intraperitoneal, intramuscular, intraventricular (e.g., intracerebroventricular), intraparenchymal, intrathecal, and intracranial injection. Where administration is by means other than injection, all known means are contemplated including administration by through the buccal, nasal, pulmonary, or rectal mucosa routes.

The terms "isolated" or "substantially purified," when applied to an agent of the invention (e.g., FTY720), denotes that the agent is essentially free of other components with which it is associated in the natural state, or during synthesis. It is preferably in a homogeneous state, although it can be in either a dry or aqueous solution. Purity and homogeneity are typically determined using analytical chemistry techniques such as polyacrylamide gel electrophoresis or high performance liquid chromatography. An agent that is the predominant species present in a preparation is substantially purified.

"Pharmaceutical composition" refers to a composition useful for administration, e.g., in a subject, particularly a human subject. A pharmaceutical composition of the invention is formulated to be compatible with its intended route of administration. Such formulations are well known in the art. Formulations that comprise therapeutically effective amounts of the FTY720 include, e.g., tablets, ampoules, capsules, sterile liquid solutions, liquid suspensions, or lyophilized versions, and optionally contain stabilizers or excipients, as described in detail herein. Lyophilized compositions are reconstituted with suitable diluents, e.g., water for injection, saline, 0.3% glycine and the like, at a level of about from 5 µg/kg of host body weight to approximately 0.07 mg/kg, 0.01 mg/kg to 1 mg/kg, 1 ng/kg to 1 mg/kg, or 1 µg/kg to 0.1 mg/kg/day, or more.

"Oral" administration refers to the delivery of the formulation via the mouth through ingestion, or via any other part of the gastrointestinal system including the esophagus or through suppository administration.

"Parenteral" administration refers to the delivery of a composition, such as a composition comprising a neurogenesis modulating agent by a route other than through the gastrointestinal tract. In particular aspects, parenteral administration may be via intravenous, subcutaneous, intramuscular or intramedullary (i.e., intrathecal) injection or infusion.

"Topical" administration refers to the application of a pharmaceutical composition to the external surface of the skin or the mucous membranes (including the surface membranes of the nose, lungs and mouth) such that the agent crosses the external surface of the skin or mucous membrane and enters the underlying tissues. Application to the mucous membrane of the mouth may also be considered a form of oral administration. Topical administration of a pharmaceutical composition can result in a targeted distribution of FTY720 to the mucous membranes and surrounding tissues. The pharmaceutical composition may also be topically applied so as to enter the bloodstream, and result in systemic distribution of FTY720.

As used herein, the term "FTY720" includes FTY720 and its derivatives as described in detail herein, but excludes S1P. Derivatives of FTY720 encompass, e.g., phosphate ester metabolites of FTY720 and pharmaceutically acceptable salts thereof, FTY720 phosphate bioisosteres, as well as FTY720 modified to further facilitate the transfer across cellular boundaries and the blood brain barrier to access NSC *in vitro* and *in vivo.* Unmodified FTY720 or FTY720P (see below) can also be used to cross-the blood brain barrier.

### FTY720 compounds of the invention

The invention encompasses compositions and methods utilizing FTY720 (2-amino-2-[2-(4-octylphenyl)ethyl]-1,3-propanediol) and its derivatives. See, e.g., Table 1; U.S. Patent 6,004,565, U.S. Patent 6,476,004, WO 99/36065, and WO 94/08943. Derivatives of FTY720 include any chemical modifications of the FTY720 molecule (see, e.g., Table 2), with the proviso that the derivative is not S1P. Non-limiting examples of derivatives include, e.g., phosphate ester metabolites of FTY720, pharmaceutically acceptable salts of FTY720, phosphate bioisosteres of FTY720, as well as compounds in which the phosphate group is modified such that the compound is facilitated in crossing cellular membranes and the blood brain barrier. Unmodified FTY720 and FTY720P (see below) can also be used to cross the blood brain barrier. Optionally, FTY720 may be pegylated to enhance its half life after administration. Methods of pegylating proteins and reagents are well known to those of skill in the art and are described, for example, in U.S. Patents 5,166,322, 5,766,897, 6,420,339 and 6,552,170.

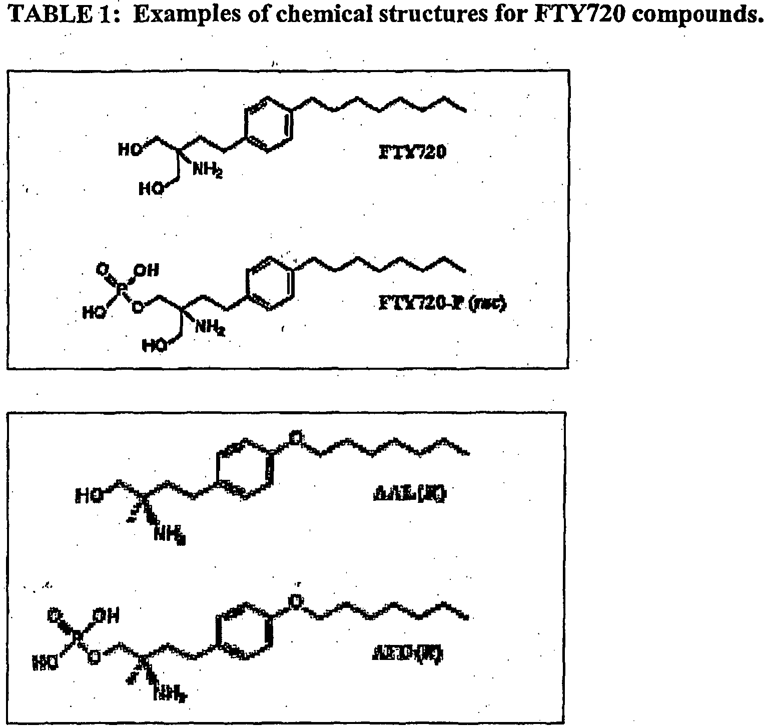

Table 1: FTY720 structures, including FTY720, FTY720P, *R*-AAL, and *R*-AFD (Brinkmann et al., 2002).

In accordance with the methods of the invention, FTY720 may be used in combination with a stabilizer, such as cyclodextrin (e.g., natural cyclodextrins, branched cyclodextrins, alkyl-cyclodextrins and hydroxyalkyl-cyclodextrins; see, e.g., EP 1050301). FTY720 also may be used with a sugar, such as a monosaccharide, disaccharide, and sugar alcohol (D-mannitol, glucose, D-xylitol, D-maltose, D-sorbitol, lactose, fructose and sucrose), which can reduce the side effects occasionally associated with FTY720 administration. In addition, FTY720 may be used in combination with one or more growth factors, such as EGF, PDGF, TGF-alpha, FGF-1, FGF-2, NGF, PACAP, and others, or with one or more anti-depressants, anti-anxiety treatments, anti-psychotic treatments, epilepsy treatments, Alzheimer's treatments, Parkinson's treatments, dopamine receptor agonists, tranquilizers, sedatives, lithium, or other therapeutics as described in detail herein.

FTY720 is currently under development by Novartis as an immunosuppressive drug. In these studies, FTY720 has been shown to efficiently prevent allograft rejection after liver and renal transplantations and exhibits a synergistic effect when given together with cyclosporin A (reviewed in Brinkmann et al, 2001) With the exception of antibodies directed towards certain T-cell epitopes, other immunosuppressive drugs used in transplantation are all poisonous substances that decrease the aggressive response of T-cells. FTY720 is a new type of immunosuppressant, which acts by redistributing lymphocytes from circulation to secondary lymphoid organs (Chiba, K. et al., 1999).

By this mechanism, FTY720 can successfully alleviate symptoms of many diseases involving autoimmunity, such as various animal models of graft vs. host disease, (e.g. Masubuchi, Y., et al., 1996), type I diabetes (Maki, T., et al., 2002; Yang, Z., 2003), rheumatoid arthritis (Matsuura, M., Imayoshi, T. & Okumoto, T., 2000; Matsuura, M., Imayoshi, T., Chiba, K. & Okumoto, T., 2000) and multiple sclerosis (MS) (Webb, M., et al., 2004; Brinkmann et al., 2002; Fujino et al., 2003). For diabetes, Yang et al. (2003) showed that FTY720 prevented autoimmune diabetes in non-obese diabetic mice. The numbers of circulating lymphocytes were significantly reduced by FTY720. In addition, the infiltration of mononuclear cells in to the islets (the hallmark of type I diabetes) was sharply diminished. FTY720 is known to induce apoptosis in various cell lines and, therefore it is also being developed for treatment of cancer. Recently, it has been reported that FTY720 is a potent inducer of apoptosis in human hepatoma cell lines, probably through downregulation of thd Akt pathway (Lee et al., 2004).

FTY720 is in Phase III studies for use as an immunosuppressant and in Phase II studies for immunologically-based treatment of MS. Owing to its amphipathic character, FTY720 exhibits good oral bioavailability (80% in rats, 60% in dogs, and 40% in monkeys). FTY720 has been shown to be metabolized primarily by CYP4F3 to the corresponding carboxylic acid. The T_{1/2} in rats after a single oral dose of 0.1 and 1 mg/kg were 18.1 and 21.6 hours, respectively. A pharmacokinetic study after a single oral administration of FTY720 at 0.3 mg/kg/day showed a T_{1/2} of 36 +/-12 hours. In a phase I study of renal transplant patients, measurement of whole blood level of FTY720 during 96 hours after a single oral dose of 0.25-3 mg showed that FTY720 is slowly absorbed with a Cₘₐₓ and AUC proportional to the dose, indicating linear pharmacokinetics.

Thus, FTY720 has been established as an active immunosuppressant and an anti-cancer/anti-proliferative agent. FTY720 is therefore a useful compound for pharmaceutical and laboratory formulations. FTY720 has been shown to be therapeutically effective and well tolerated when orally administered for immunosuppression. The only observed side effect has been mild and transient bradycardia. As expected by its mode of action, FTY720 is also associated with a decrease in peripheral lymphocyte numbers (see, e.g., Tedesco-Silva H, et al., 2004). Yet, a broad range of doses of FTY720 can be used safely for the treatment methods described herein. In rat models, the LD₅₀ has been calculated as high as 300-600 mg/kg (IDDB; available at world wide web.iddb.org/). In dog models, no deaths were observed below dosages of 200 mg/kg (IDDB). Primates were treated with 3 mg/kg with no observed toxicity (IDDB). In PI renal transplant patients, administration of 0.25-3.5 mg (single dose) of drug produced no serious adverse events. Thus, in accordance with particular aspects of the invention, FTY720 dosage can range from approximately 5 µg/kg to approximately 0.07 mg/kg for the treatment of human subjects. In addition, unmodified FTY720 and FTY720P are able to cross the blood brain barrier.

Surprisingly, the experiments of the invention show that the known immunosuppressant and anti-cancer drug FTY720 acts to stimulate activity of NSCs/NPCs, and therefore can be used to stimulate neurogenesis *in vivo* and cultivate NSC *in vitro.*

### FTY720 and S1P receptors

FTY720 shares some structural characteristics with the endogenous lysophospholipid sphingosine including a lipophilic tail, a 2-amino group, and a phosphate head group (Table 2). Sphingosine and FTY720 are phosphorylated *in vivo* resulting in sphingosine-1-phosphate (S1P) and FTY720P, respectively. Both S1P and FTY720P are ligands for a group of G protein coupled receptors, the S1P receptors (S1PRs) named S1P₁-S1P₅. These were formerly called EDG receptors (EDG₁ = S1P₁; EDG₃ = S1P₃; EDG₅ = S1P₂; EDG₆ = S1P₄; EDG₈ = S1P₅).

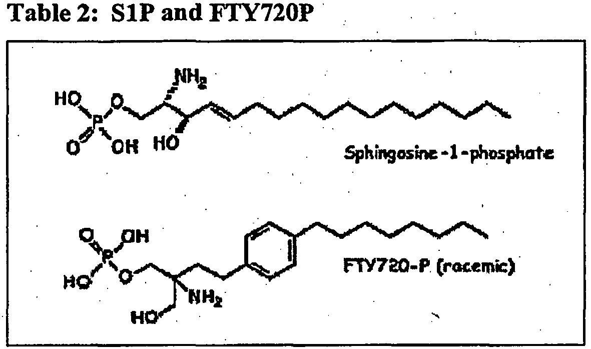

Table 2: The structure of S1P (above) and FTY720P (below).

As shown herein, mRNA expression of all S1P receptors in neurogenic areas or relevant cells/tissue, in particular S1P₁ and S1P₅ are highly and selectively expressed in neurogenic areas (lateral ventricular wall (LVW) or hippocampus). In addition, S1P receptors are expressed in the lateral ventricle wall (LVW) of the adult mouse brain and adult mouse NSC cultured in *vitro*. Moreover, S1P₁ is expressed specifically in the subventricular zone (SVZ) of the LVW and S1PR₅ is expressed in the dentate gyrus of the hippocampus.

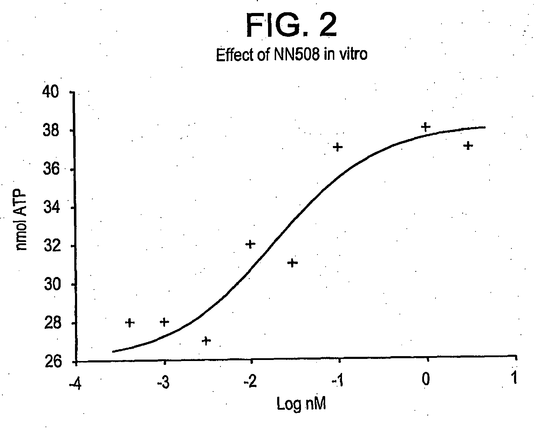

FTY720 and its phosphorylated metabolite have been characterized as high affinity ligands of at least two S1P receptors expressed in the brain (Mandala et al, 2002). The lowest EC₅₀ values reported for FTY720 is for S1P₁ and S1P₅. Picomolar affinities have been reported for its phosphorylated form, FTY720P (see Table 3). However, FTY720 shows distinct and unexpected binding and activity as compared to S1P. FTY720P binds with higher affinity to S1P₁ receptor, but with lower affinity to S1P₃ receptor, as compared to S1P (Table 3). FTY720P binds to all S1PRs except S1P₂, while S1P binds to all S1PRs except S1P₄ (Table 3; reviewed in Fujino, M., et al., 2003). In addition, S1P has been utilized in neural progenitor cells at concentrations of 100 nM to 3 µM (Harada et al., 2001), while this invention demonstrates that FTY720 is effective in neural stem cells at EC₅₀ value of 0.02 nM to 0.04 nM (FIG. 2). Thus, FTY720 is active at a concentration of 0.001 nM to 0.05 nM in target NSC. These data taken together with the *in situ* hybridization results disclosed herein below suggest that FTY720 mediate proliferation via S1P₁ and/or S1P₅. The differences in the binding profiles of S1P and FTY720 may explain the significantly lower toxicity and incidents of side effects associated with FTY720.

**TABLE 3: FTY720P affinities for S1PRs.**

| **Potency (EC₅₀)*** | **S1P₁** | **S1P₂** | **S1P₃** | **S1P₄** | **S1P₅** |
|---|---|---|---|---|---|
| **FTY720P^{##}** | 8.2 | - | 8.4 | 7.2 | 8.2 |
| **FTY720P^{###}** | 1.4 | >10 000 | 580 | 43 | 37 |
| **Binding (IC₅₀)**** | | | | | |
| **FTY720P^{#}** | 0.21 +/- 0.17 | >10,000 | 5.0 +/- 2.7 | 5.9 +/- 2.3 | 0.59 +/- 0.27 |
| **S1P^{#}** | 0.47 -+/ 0.34 | 0.31 +/- 0.02 | 0.17 +/- 0.05 | 95 +/- 25 | 0.61 +/- 0.39 |
| FTY720 ^{#} | 300 +/- 51 | >10,000 | >10,000 | >5000 | 2623 +/- 317 |

| | | | | | |
|---|---|---|---|---|---|
| Table 3: Binding data for S1P receptors. * [γ-³⁵S]GTPγS binding assay (nM). **Competition of S1³³P binding to membranes of stably transfected CHO cells expressing the indicated S1P receptor (nM). ^{#} Mandala et al., 2002; ^{##} Brinkmann et al., 2002; ^{###} Novartis data. | | | | | |

### Cloning and expression of S1PRs

All S1PRs are encoded by a single exon. S1P₁ was the first S1PR to be cloned. It was originally discovered as a transcript induced during endothelial cell differentiation. This gave rise to the former name, EDG (endothelial differentiation gene). Both human and mouse S1P₁ include 382 amino acids with apparent molecular masses of ∼43 kDa. S1P₂ (human 353 amino acids; mouse 352 amino acids) was later isolated from rat brain and rat vascular smooth muscle cell, whereas S1P₃ was isolated from a human genomic library. S1P₄ was cloned from *in vitro* differentiated human and mouse dendritic cells. S1P₅ was shown to correspond to a gene called *nrg-1,* which was cloned from a PC12 cell cDNA library. When the first S1PR was deorphanized, it was discovered that S1P₁ was activated by S1P. Sequence analysis revealed that the S1PRs exhibit ∼20% amino acid sequence identity with cannabinoid receptors and ∼30% identity with lysophosphatidic acid receptors (LPA₁₋₃, formerly called EDG_{2,4,7}).

Mouse S1P₁ is expressed primarily in the brain, heart, lung, and spleen, but also to a lesser extent in kidney, thymus, and muscle. S1P₁ displays a marked expression in the CNS together with S1P₅, which was found to be expressed in the brain. S1P₂ and S1P₃ are closely related (92% sequence identity) and share similar tissue distribution. These receptors are expressed in heart and lung, but also kidney, liver, thymus, spleen, testis and brain. S1P₄ is exclusively expressed in lymphoid tissue (Graler, et al., 2002). and S1P₅ is exclusively expressed in the brain (Glickman et al., 1999).

Chae et al. recently used a β-galactosidase reporter gene expression system, where β-galactosidase is knocked in to the S1P₁ locus to detect its expression in more detail. In the adult mouse brain, S1P₁ was found to be expressed by purkinje cells, neuronal cell bodies as well as by astrocytes (Chae, et al., 2004). In a study by Beer et al, the distribution of S1PR mRNA within the nervous system was investigated. They showed that S1P₃ is confined to neuronal cells. In line with earlier studies, S1P₄ mRNA was not detected at all in the CNS (Beer, et al., 2000). S1P₃ expression within the mouse brain was found to be restricted to the choroid plexus of the fourth ventricle, scattered cells of the diencephalons (McGiffert, et al., 2002). By Northern blot analysis and EST expression profiling, it was demonstrated that rat S1P₅ expression is particularly abundant in lower brain regions including midbrain, pons, medulla and spinal cord (Glickman, et al., 1999). Human S1P₅ expression has also been localized to the brain, lung, spleen, and peripheral blood leukocytes (Im, et al., 2001). In addition, S1P₅ has been localized to the corpus callosum, hippocampus (fimbra of) and white matter (Im, et al., 2000).

FTY720 has been reported to induce apoptosis in lymphocytes and other cell lines. This is associated with a rapid increase of intracellular Ca²⁺ levels and is dependent on phospholipase C (Shinomiya, et al., 1997) in PTX independent way. This mechanism may bypass the S1P₁ receptor. Additional information regarding S IPRs and their putative roles is generally available (for review, see Toman, et al., 2002).

### Diseases and disorders treated by the invention

The invention encompasses the use of FTY720 or certain derivatives thereof in the manufacture of a medicament for treating a neurodegenerative disease; a neurological disease or a psychiatric disease, with the proviso that the disorder is not multiple sclerosis (MS) or a demyelinating disease such as Guillain Barre syndrome.

It should be noted that MS can be distinguished from other motor neuron diseases (e.g., amyotrophic lateral sclerosis, or ALS) by basic differences in the pathology and the progression of this pathology (see, e.g., C. Plank, The Center for Neurologic Study available at world wide web.ensonline.org). The principle characteristic in the pathology of ALS is loss of motor nerve cells in the anterior horns of the spinal cord and in the motor nuclei of the brain stem. By comparison, MS is primarily a disease of myelin, not nerve cells. The myelin surrounds the axons, or the long process of the nerve cell. Since myelin occurs throughout the nervous system, lesions can be and typically are at multiple sites. The disease, however, affects only central myelin, not the myelin of peripheral nerves. The other elements of central nervous tissue are relatively unaffected in MS, including the actual nerve cells, their processes and axis cylinders, and the supporting tissue. The destruction of the myelin covering the axon does not result in significant retrograde degeneration of the axons themselves. That is, nerve cells, surprisingly, do not show significant evidence of destruction in MS. Another striking feature of MS is autoimmunity, which has been targeted by treatment with FTY720.

In accordance with the invention, non-limiting examples of nervous system disorders include, for example, at least the following: neurodegenerative disorders, neural stem cell disorders, neural progenitor disorders, ischemic disorders, neurological traumas and injuries, affective disorders, neuropsychiatric disorders, degenerative diseases of the retina, retinal injury and trauma, and learning and memory disorders. Also included are schizophrenia and other psychoses, lissencephaly syndrome, depression, bipolar depression, bipolar disorder, anxiety syndromes, anxiety disorders, phobias, stress and related syndromes, cognitive function disorders, aggression, drug and alcohol abuse, obsessive compulsive behavior syndromes, seasonal mood disorder, borderline personality disorder, cerebral palsy. In further aspects of the invention, the disorder of the nervous system includes, at least, dementia, epilepsy, injury related to epilepsy, and temporal lobe epilepsy. Also included are spinal cord injury, brain injury, brain surgery, trauma related brain injury, trauma related to spinal cord injury, brain injury related to cancer treatment, spinal cord injury related to cancer treatment, brain injury related to infection, brain injury related to inflammation, spinal cord injury related to infection, spinal cord injury related to inflammation, brain injury related to environmental toxin, spinal cord injury related to environmental toxin, autism, attention deficit disorders, narcolepsy, sleep disorders, and cognitive disorders.

In specific aspects of the invention, the disorder of the nervous system includes, at least, Parkinson's disease (shaking palsy), including primary Parkinson's disease, secondary parkinsonism, and postencephalitic parkinsonism; drug-induced movement disorders, including parkinsonism, acute dystonia, tardive dyskinesia, and neuroleptic malignant syndrome; Huntington's disease (Huntington's chorea; chronic progressive chorea; hereditary chorea); delirium (acute confusional state); dementia; Alzheimer's disease; non-Alzheimer's dementias, including Lewy body dementia, vascular dementia, Binswanger's dementia (subcortical arteriosclerotic encephalopathy), dementia pugilistica, normal-pressure hydrocephalus, general paresis, frontotemporal dementia, multi-infarct dementia, and AIDS dementia; age-associated memory impairment (AAMI); amnesias, such as retrograde, anterograde, global, modality specific, transient, stable, and progressive amnesias, and posttraumatic amnesias, and Korsakoff's disease.

Other specific disorders include idiopathic orthostatic hypotension, Shy-Drager syndrome, progressive supranuclear palsy (Steele-Richardson-Olszewski syndrome); structural lesions of the cerebellum, such as those associated with infarcts, hemorrhages, or tumors; spinocerebellar degenerations such as those associated with Friedreich's ataxia, abetalipoproteinemia (e.g., Bassen-Kornzweig syndrome, vitamin E deficiency), Refsum's disease (phytanic acid storage disease), cerebellar ataxias, multiple systems atrophy (olivopontocerebellar atrophy), ataxia-telangiectasia, and mitochondrial multisystem disorders; acute disseminated encephalomyelitis (postinfectious encephalomyelitis); adrenoleukodystrophy and adrenomyeloneuropathy; Leber's hereditary optic atrophy; HTLV-associated myelopathy; motor neuron disorders such as amyotrophic lateral sclerosis, progressive bulbar palsy, progressive muscular atrophy, primary lateral sclerosis and progressive pseudobulbar palsy, and spinal muscular atrophies such as type I spinal muscular atrophy (Werdnig-Hoffmann disease), type II (intermediate) spinal muscular atrophy, type III spinal muscular atrophy (WohlfartKugelberg-Welander disease), and type IV spinal muscular atrophy.

Additional specific disorders include plexus disorders such as plexopathy and acute brachial neuritis (neuralgic amyotrophy); peripheral neuropathies such as mononeuropathies, multiple mononeuropathies, and polyneuropathies, including lunar nerve palsy, carpal tunnel syndrome, peroneal nerve palsy, radial nerve palsy, GuillainBarre syndrome, chronic relapsing polyneuropathy, hereditary motor and sensory neuropathy, e.g., types I and II (Charcot-Marie-Tooth disease, peroneal muscular atrophy), and type III (hypertrophic interstitial neuropathy, Dejerine-Sottas disease); disorders of neuromuscular transmission, such as myasthenia gravis; neuroophthalmologic disorders such as Homer's syndrome, intemuclear ophthahnoplegia, gaze palsies, and Parinaud's syndrome; cranial nerve palsies, trigeminal neuralgia (Tic Douloureux); Bell's palsy; and glossopharyngeal neuralgia; radiation-induced injury of the nervous system; chemotherapy-induced neuropathy (e.g., encephalopathy); taxol neuropathy; vincristine neuropathy; diabetic neuropathy; autonomic neuropathies; polyneuropathie;, and mononeuropathies; and ischemic syndromes such as transient ischemic attacks, subclavian steal syndrome, drop attacks, ischemic stroke, spinal ischemia, hemorrhagic stroke, and brain infarction.

Other nervous system disorders disclosed in co-pending U.S. Applications Nos. 09/998,861, 10/246,091, 10/291,290, 10/291,171 and 10/429,062.

### Pharmaceutical compositions of the invention

We disclose a composition comprising FTY720 for use in treating a nervous system disorder by stimulating NSC activity and thereby replacing damaged or missing neurons in the nervous system. In accordance with such uses, FTY720 is provided in a suitable formulation through a suitable route of administration so as to modulate NSC or NPC activity *in vivo.*

In one aspect, the invention includes regenerative uses for treating one or more symptoms of a neurodegenerative disease in a subject by administering an FTY720 composition to stimulate neurogenesis (i.e., cell growth, proliferation, migration, survival and/or differentiation) of ependymal cells and subventricular zone to obtain the desired neural phenotype. As examples, FTY720 can be used to increase neurogenesis in one or more loci, e.g., in regions where cells are damaged or missing or in undamaged regions. *In vivo* stimulation of ependymal stem cells is accomplished by locally administering FTY720 to the cells in an appropriate formulation. By increasing neurogenesis, damaged or missing neurons can be replaced in order to enhance brain function in diseased states.

In a particular aspect, the invention includes uses wherein an FTY720 composition is administered to a mammal. The term "mammal" refers to any mammal classified as a mammal, including humans, cows, horses, dogs, sheep, cats, rabbits, mice, and rats. In a preferred aspect, the mammal is a human.

Encompassed by the invention are pharmaceutical compositions that are useful for the treatment of nervous system disorders. For example, the compositions include a FTY720 compound, which can be administered alone or in combination with the systemic or local co-administration of one or more additional agents. Such agents include growth factors, preservatives, ventricle wall permeability increasing factors, stem cell mitogens, survival factors, glial lineage preventing agents, anti-apoptotic agents, anti-stress medications, neuroprotectants, and anti-pyrogenics. The pharmaceutical compositions preferentially treat nervous system diseases by stimulating cells (e.g., ependymal cells and subventricular zone cells) to grow, proliferate, survive, migrate, or diffdrentiate into the desired neural phenotype, targeting loci where cells areclamaged or missing.

Use of FTY720 and its derivatives in treating a nervous system disorder is also provided This use comprises administering to the subject an effective amount of a pharmaceutical composition that includes FTY720 (1) alone fit a dosage range of 0.001 ng/kg/day to 10 mg/kg/day, preferably in a dosage range of 0.01 ng/kg/day to 5 mg/kg/day, preferably in a dosage range of 0.1 ng/kg/day to 1 mg/kg/day, preferably in a dosage range of 100 ng/kg/day to 1 mg/kg/day, most preferably at 1 ng/kg/day to 1 mg/kg/day or 1 lig/kg/day to 0.1 mg/kg/day, (2) in a combination with a ventricle wall permeability increasing factor, or (3) in combination with a locally or systemically co-administered agent.

Examples of routes of administration include oral, subcutaneous, intraperitoneal, intramuscular, intraventricular (e.g., intracerebroventricular), intraparenchymal, intrathecal, intracranial, buccal, mucosal, nasal, and rectal routes. A parenteral preparation can be formulated for delivery via ampoules, disposable syringes or multiple dose vials made of glass or plastic In addition, the pharmaceutical composition and neurogenesis modulating agent of the invention may be delivered as an eye drop, eye ointment, or nose drop. In case that the composition of the invention is used in the form of an eye drop or a nasal drop, the solvent employed includes a sterile distilled water or, in particular a distilled water for injection. The concentration of the active compound usually ranges from 0.01 to 2.0 w/v%, and may be increased or decreased depending on the aim of use. The eye drop or a nasal drop may further contain various additives such as a buffer, an isotonic agent, a solubilizing agent, a preservative, a viscosity-increasing agent, a chelating agent, a pH adjustor, or an aromatic.

For they eye drop and nasal drop, the preservative may include, for example, a quaternary ammonium salt such as benzalkonium chloride, benzethonium chloride or cetyl pyridinium chloride, a parahydroxybenzoic acid ester such as methyl parahydroxybenzoate, ethyl parahydroxybenzoate, propyl parahydroxybenzoate or butyl parahydroxybenzoate, benzyl alcohol, phenethyl alcohol, sorbic acid or a salt thereof, thimerosal, chlorobutanol, sodium dehydroacetate, methylparaben or propylparaben. The viscosity-increasing agent may include, for example, polyvinylpyrrolidone, hydroxyethylcellulose, hydroxypropylcellulose, methylcellulose, hydroxypropyl-methylcellulose, or carboxymethylcellulose or a salt thereof. The chelating agent may include disodium edetate or citric acid and the like. The pH adjustor may include hydrochloric acid, citric acid, phosphoric acid, acetic acid, tartaric acid, sodium hydroxide, potassium hydroxide, sodium carbonate or sodium bicarbonate and the like. The aromatic may include 1-menthol, borneol, a camphor (e.g., DL-camphor), eucalyptus oil, and the like. The eye drop and nasal drop can typically be adjusted to about pH 4.0 to about pH 8.5.

Additionally, the pharmaceutical composition and neurogenesis modulating agent of the invention may be delivered by nasal or pulmonary administration. The respiratory delivery of aerosolized therapeutics is described in a number of references (see, e.g., Gansslen 1925; Laube et al. 1993; Elliott et al. 1987; Wigley et al. 1971; Colthorpe et al. 1992; Govinda 1959; Hastings et al. 1992; Nagano et al. 1985; Sakr 1992; and Yoshida et al. 1987). Pulmonary delivery of dry powder therapeutics is described in U.S. Pat. No. 5,254,330. A metered dose inhaler is described, e.g., in Lee and Sciara 1976. The intrabronchial administration of recombinant insulin is briefly described in Schlutiter et al. 1984; and Kohler et al. 1987. Intranasal and respiratory delivery of a variety of agents is described in U.S. Pat. No. 5,011,678 and Nagai et al. 1984.

Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi.

The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include one or more isotonic agents, for example, sugars, polyalcohols such as manitol, sorbitol, and sodium chloride in the composition. Prolonged absorption of the injectable compositions can be brought about by including in the composition an agent which delays absorption, for example, aluminum monostearate, and gelatin.

Sterile injectable solutions can be prepared by incorporating FTY720 in the required amount in an appropriate solvent with one or a combination of ingredients enumerated above, as required, followed by filtered sterilization. Generally, dispersions are prepared by incorporating FTY720 into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, FTY720 can be incorporated with excipients and used in the form of tablets, troches, or capsules. Oral compositions can also be prepared using a fluid carrier for use as a mouthwash, wherein FTY720 in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compositions of the invention can be delivered in an aerosolized form to the human respiratory system (e.g., nasal, oral, tracheal, bronchial, and alveolar sites) using inhalers or nebulizers. For example, metered dose inhalers, dry powder inhalers, or aqueous-based inhalers can be used.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or nasal suppositories. For transdermal administration, the FTY720 compositions of the invention can be formulated into ointments, salves, gels, or creams, other means for external application as generally known in the art (see, e.g., EP 0812588). The FTY720 compositions can also be prepared in the form of nasal drops or sprays, or suppositories (e.g., with conventional suppository bases such as cocoa butter and other glycerides) or retention enemas for rectal delivery.

In one embodiment, FTY720 is prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to cells with monoclonal antibodies) can also be used as pharmaceutically acceptable carriers. These can be prepared according to methods known to those skilled in the art, for example, as described in U.S. Pat. No. 4,522,811.

Additionally, suspensions of the active compounds may be prepared as appropriate oily injection suspensions. Suitable lipophilic solvents or vehicles include fatty oils, such as sesame oil, or synthetic fatty acid esters, such as ethyl oleate, triglycerides, or liposomes. Non-lipid polycationic amino polymers may also be used for delivery. Optionally, the suspension may also contain suitable stabilizers or agents to increase the solubility of the compounds and allow for the preparation of highly concentrated solutions.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of FTY720 calculated to produce the desired therapeutic effect in association with the required pharmaceutical carrier. The specification for the dosage unit forms of the invention are dictated by and directly dependent on the unique characteristics of FTY720 and the particular therapeutic effect to be achieved, and the limitations inherent in the art of compounding such an active compound for the treatment of individuals.

The pharmaceutical compositions can be included in a container, pack, or dispenser together with instructions for administration.

In another embodiments, the reagent is administered in a composition comprising at least 90% pure FTY720.

Preferably FTY720 is formulated in a medium providing maximum stability and the least formulation-related side effects. In addition to FTY720, the composition of the invention will typically include one or more protein carrier, buffer, isotonic salt, and stabilizer.

In some instances, FTY720 can be administered by a surgical procedure implanting a catheter coupled to a pump device. The pump device can also be implanted or be extracorporally positioned. Administration of the reagent can be in intermittent pulses or as a continuous infusion. Devices for injection to discrete areas of the brain are known in the art (see, e.g., U.S. Patent Nos. 6,042,579; 5,832,932; and 4,692,147).

) FTY720 compositions can be administered in any conventional form for administration of a lipid. FTY720 can be administered in any manner known in the art in which it may either pass through or by-pass the blood-brain barrier. Methods for enhancing passage through the blood-brain barrier include minimizing the size of the factor, providing hydrophobic factors which may pass through more easily, conjugating the protein reagent or other agent to a carrier molecule that has a substantial permeability coefficient across the blood brain barrier (see, e.g., U.S. Patent 5,670,477).

Reagents, derivatives, and co-administered agents can be incorporated into pharmaceutical compositions suitable for administration. Such compositions can typically comprise FTY720 and a pharmaceutically acceptable carrier. As used herein, "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated.

Supplementary active compounds can also be incorporated into the compositions. Modifications can be made to FTY720 to affect solubility or clearance of the molecule. Peptidic molecules may also be synthesized with D-amino acids to increase resistance to enzymatic degradation. In some cases, the composition can be co-administered with one or more solubilizing agents, preservatives, and permeation enhancing agents. Examples of pharmaceutically acceptable carriers include lactose, glucose, sucrose, sorbitol, mannitol, corn starch, crystalline cellulose, gum arabic, calcium phosphate, alginates, calcium silicate, microcrystalline cellulose, polyvinyl pyrrolidone, tragacanth gum, gelatin, syrum, methyl cellulose, carboxymethyl cellulose, methylhydroxybenzoic acid esters, propylhydroxybenzoic acid esters, talc, magnesium stearates, inert polymers, water and mineral oils.

For example, the composition can include a preservative or a carrier such as proteins, carbohydrates, and compounds to increase the density of the pharmaceutical composition. The composition can also include isotonic salts and redox-control agents.

In some embodiments, the composition administered includes the reagent and one or more agents that increase the permeability of the ventricle wall, e.g. "ventricle wall permeability enhancers." Such a composition can help an injected composition penetrate deeper than the ventricle wall. Examples of suitable ventricle wall permeability enhancers include, for example, liposomes, VEGF (vascular endothelial growth factor), IL-s, TNFα, polyoxyethylene, polyoxyethylene ethers of fatty acids, sorbitan monooleate, sorbitan monolaurate, polyoxyethylene monolaurate, polyoxyethylene sorbitan monolaurate, fusidic acid and derivatives thereof, EDTA, disodium EDTA, cholic acid and derivatives, deoxycholic acid, glycocholic acid, glycodeoxycholic acid, taurocholic acid, taurodeoxycholic acid, sodium cholate, sodium glycocholate, glycocholate, sodium deoxycholate, sodium taurocholate, sodium glycodeoxycholate, sodium taurodeoxycholate, chenodeoxycholic acid, urosdeoxycholic acid, saponins, glycyrrhizic acid, ammonium glycyrrhizide, decamethonium, decamethonium bromide, dodecyltrimethylammonium bromide, and dimethyl-β-cyclodextrin or other cyclodextrins.

### Therapeutic methods and uses of the invention

We also disclose FTY720 for use in stimulating the activity of NSC for therapeutic purposes. The uses of the invention can be used for modifying and manipulating NSC *in vivo* to allow treatment of various nervous system diseases, disorders, and injuries that affect neural pathways. In one aspect, the uses of the invention involve contacting NSC with a composition comprising FTY720 in an amount sufficient to stimulate growth, proliferation, differentiation, or survival of the NSC. In a particular aspect, FTY720 stimulates the activity of the S1PR signaling pathway. The uses disclosed herein can be performed *in vitro* (e.g., by culturing the cell with FTY720) or, alternatively, *in vivo* (e.g., by administering FTY720 to a subject). Thus, we disclose uses for treating an individual afflicted with a disorder, specifically a nervous system disorder. The uses are particularly useful for disorders characterized by aberrant cell proliferation, differentiation, migration, or survival.

In various aspects of the invention, suitable *in vitro* or *in vivo* assays can be performed to determine the effect of FTY720 on target tissue and whether its administration is indicated for treatment of the particular disorder. For example, *in vitro* assays may be performed with representative stem cells or newly differentiated cells involved in the subject's disorder, to determine if FTY720 exerts the desired effect upon the cell type(s). FTY720 compositions for use in therapy may be tested in suitable animal model systems including, but not limited to rats, mice, chicken, cows, monkeys, rabbits, and the live, prior to testing in human subjects. Similarly, for *in vivo* testing, any of the animal model system known in the art may be used prior to administration to human subjects.

The disclosed uses for treating take advantage of the NSC located in the tissues lining ventricles of non-embryonic (e.g., adult) brains. The ventricular system is found in nearly all brain regions and thus allows easier access to the affected areas. In accordance with these uses, therapy for nervous system diseases can be tailored so that stem cells surrounding ventricles near the affected region are manipulated or modified as needed. NSC activity can be altered *in vivo* by exposing the cells to a composition comprising FTY720.

In one aspect, a device can be implanted to administers the FTY720 composition to the ventricle and thus, to the neural stem cells. Alternatively, a cannula attached to an osmotic pump may be used to deliver the composition. Alternatively, the composition may be injected directly into the ventricles. The neural stem cell progeny can then migrate into regions that have been damaged as a result of injury or disease. The close proximity of the ventricles to many brain regions would allow for the diffusion of FTY720 into stem cells or their progeny.

In an additional aspect, a FTY720 composition of the invention can be administered locally, as described herein, in combination with an agent administered locally or systemically. Such agents include, for example, one or more growth factors, stem cell mitogens, survival factors, glial-lineage preventing agents, anti-apoptotic agents, anti-stress medications, neuroprotectants, and anti-pyrogenics, or any combination thereof. The agent can be administered before, during, or after administration of FTY720.

Administration may be by any means. Preferably, FTY720 compositions are administered systemically. Oral administration and injection are particularly preferred. The administration may be by infusion. The delivery may be subcutaneously, intraperitoneally, intramusblularly, intraventricularly (e.g., intracerebroventricularly), intraparenchymally, intrathecally or intracranially. As another example, the administration may be made orally or nasally. Administration may be carried out via inhalation (e.g., in an aerosol, for example, a dry powder or aqueous-based spray, or a nebulizer), peptide fusion, or micelle delivery.

For treatment of Huntington's disease, Alzheimer's disease, Parkinson's disease, and other neurological disorders affecting primarily the forebrain, FTY720 can be administered alone or with an additional agent or agents delivered to the ventricles of the forebrain to affect in *vivo* modification or manipulation of NSC. For example, Parkinson's disease is the result of low levels of dopamine in the brain, particularly the striatum. It is therefore advantageous to induce a patient's own quiescent stem cells to begin to divide *in vivo* and to induce the progeny of these cells to differentiate into dopaminergic cells in the affected region of the striatum, thus locally raising the levels of dopamine.

Normally the cell bodies of dopaminergic neurons are located in the substantia nigra and adjacent regions of the mesencephalon, with the axons projecting to the striatum. The methods and compositions of the invention provide an alternative to the use of drugs and the controversial use of large quantities of embryonic tissue for treatment of Parkinson's disease. Dopamine cells can be generated in the striatum by the administration of a composition comprising FTY720 to the lateral ventricle.

For the treatment of amyotrophic lateral sclerosis or other motor neuron diseases, excluding multiple sclerosis, FTY720 can be delivered to the systemically, or e.g., to the central canal, alone or with an additional agent or agents.

In addition to treating nervous system tissue immediately surrounding a ventricle, FTY720 can be administered to the lumbar cistern for circulation throughout the nervous system (e.g., CNS), alone or with an additional agent or agents.

In other aspects, neuroprotectants can also be co-administered systemically or locally before, during, and/or after infusion of FTY720. Neuroprotectants include antioxidants (agents with reducing activity, e.g., selenium, vitamin E, vitamin C, glutathione, cysteine, flavinoids, quinolines, enzymes with reducing activity, etc), Cachannel modulators, Na-channel modulators, glutamate receptor modulators, serotonin receptor agonists, phospholipids, unsaturated- and polyunsaturated fatty acids, estrogens and selective estrogen receptor modulators (SERMS), progestins, thyroid hormone and thyroid hormone-mimicking compounds, cyclosporin A and derivatives, thalidomide and derivatives, methylxanthines, MAO inhibitors; serotonin-, noradrenaline and dopamine uptake blockers; dopamine agonists, L-DOPA, nicotine and derivatives, and NO synthase modulators.

Certain FTY720 compositions of the invention may be pyrogenic following intravenous injection (Am. J. Physiol. Regul. Integr. Comp. Physiol. 2000 278:R1275-81). Thus, in some aspects of the invention, antipyrogenic agents like cox2 inhibitors, indomethacin, salisylic acid derivatives, and other general anti-inflammatory/antipyrogenic compounds can be systemically or locally administered before, during, and/or after administration of the FTY720 composition.

In another aspect of the invention, anti-apoptotic agents including caspase inhibitors and agents useful for antisense-modulation of apoptotic enzymes and factors can be administered before, during, or after administration of FTY720.

Stress syndromes lower neurogenesis, therefore in some aspects, it may be desirable to treat a subject with anti-stress medications such as, e.g., anti-glucocorticoids (e.g., RU486) and beta-blockers, administered systemically or locally before, during and/or after administration of FTY720.

Methods for preparing FTY720 dosage forms are known, or will be apparent, to those skilled in this art. The amount of FTY720 to be administered will depend upon the exact size and condition of the subject, but will be from, e.g., 1 ng to 1 mg, 1 µg to 0.1 mg, 1 mg to 100 mg, or preferably 0.3 mg to 10 mg in a volume of 0.001 ml to 10 ml. The duration of treatment and time period of administration of reagent will also vary according to the size and condition of the subject, the severity of the illness and the specific composition and method being used.

The effectiveness of each of the foregoing methods for treating a subject with a nervous system disorder can be assessed by any known standardized test for evaluating the disorder.

### EXAMPLES

The examples are presented in order to more fully illustrate the preferred embodiments of the invention. These examples should in no way be construed as limiting the scope of the invention, as encompassed by the appended claims.

The experimental data shown herein demonstrate that FTY720 has a positive effect on modulating neurogenesis, as shown by the proliferation of NSC grown *in vitro.*

### EXAMPLE 1: Neurosphere cultures

The anterior lateral wall of the lateral ventricle of 5-6 week old mice was enzymatically dissociated at 37°C for 20 min in 0.8 mg/ml hyaluronidase and 0.5 mg/ml trypsin in DMEM containing 4.5 mg/ml glucose and 80 units/ml DNase. The cells were gently triturated and mixed with three volumes of Neurosphere medium (DMEM/F12, B27 supplement, 125 mM HEPES Ph 7.4) containing 20 ng/ml EGF (unless otherwise stated), 100 units/ml penicillin, and 100 µg/ml streptomycin. After passing through a 70 µm strainer, the cells were pelleted at 160 x g for 5 min. The supernatant was subsequently removed and the cells resuspended in Neurosphere medium supplemented as above, plated out in culture dishes and incubated at 37°C. Neurosphere cultures were ready to be split approximately 7 days after plating.

To split the neurosphere cultures, neurospheres were collected by centrifugation at 160 x g for 5 min. The conditioned supernatant (conditioned medium) was removed and saved. The neurospheres were resuspended in 0.5 ml Trypsin/EDTA in HBSS (1 x), incubated at 37°C for 2 min, and triturated gently to aid dissociation. Following a further 3 min incubation at 37°C and trituration, 3 volumes of ice cold NSPH-media-EGF were added to stop further trypsin activity. The cells were pelleted at 220 x g for 4 min, and resuspended in a 1:1 mixture of fresh Neurosphere medium and conditioned medium. EGF was supplemented to 20 ng/ml and the culture plated out and incubated at 37°C.

### EXAMPLE 2: RT-PCR analysis

Neurospheres were prepared from the LVW as stated above. Three days after the first split, the neurospheres were harvested and total RNA was isolated using QIAGEN's RNeasy Mini Kit according to the manufacturer's instructions. LVW and ROB total RNA was prepared in identical fashion to that of neurosphere total RNA. Prior to the RT-PCR, total RNA was DNase (Ambion) treated (1 unit 5 µg total RNA) at 37°C for 15 min, followed by heat inactivation at 75°C for 10 min. Invitrogen's One-Step RT-PCR Kit was used to detect the presence of mRNA corresponding to the eight EDG/S1P receptors. Briefly, 12.5 ng of total RNA was used in each reaction, with an annealing temperature of 58°C. To further ensure that genomic contamination of the total RNA did not give rise to false positive results, an identical reaction with Taq polymerase alone was run in parallel with the experimental RT-PCR. The reactions were electrophoresed on a 1.0% agarose gel containing ethidium bromide and bands were visualized under UV light. Bands corresponding to the estimated length of PCR products of the desired genes were cloned into the cloning vector pGEM-Teasy. Constructs were sequenced to verify their identity. Primer sequences are shown below.

| Receptor | Primer | Band size (bp) |
|---|---|---|
| Edg1/S1P₁ | Fw: aaaaccaagaagttccaccggccc (SEQ ID NO:1) | 639 |
| | Rev: cgccttgcagcccacatctaacagt (SEQ ID NO:2) | |
| Edg2 | Fw: cagctgcctctacttccagccctgtaattt (SEQ ID NO:3) | 509 |
| | Rev: gatgactacaatcaccaccaccacgcg a (SEQ ID NO:4) | |
| Edq3/S1P₃ | Fw: tttcatcggcaacttggctctctgc (SEQ ID NO:5) | 635 |
| | Rev: ggacagccagcatgatgaaccactg (SEQ ID NO:6) | |
| Edg4 | Fw: atgggccagtgctactacaacgagacca (SEQ ID NO:7) | 509 |
| | Rev: cagaggcagtgccagaagtgtgcaggta (SEQ ID NO:8) | |
| Edg5/S1P₂ | Fw: ggccttcgtggccaacaccttact (SEQ ID NO:9) | 629 |
| | Rev: cccggctacgccacgtatagatgac (SEQ ID NO:10) | |
| Edg6/S1P₄ | Fw: atgaacatcagtacctggtccacgctgg (SEQ ID NO:11) | 513 |
| | Rev: gcacagaccgatgcagccatacacac (SEQ ID NO:12) | |
| Edg7 | Fw: tgaatgagtgtcactatgacaagcgcatgg (SEQ ID NO:13) | 515 |
| | Rev: gttgcagaggcaattccatcccagc (SEQ ID NO:14) | |
| Edg8/S1P₅ | Fw: cggcgccggtgagtgaggttattgt (SEQ ID NO:15) | 514 |
| | Rev: aggcgtcctaagcagttccagccca (SEQ ID NO:16) | |
| Actin | Fw: atggatgacgatatcgctgcgctgg (SEQ ID NO:17) | 360 |
| | Rev: ggtcatcttttcacggttggccttagggt (SEQ ID NO: 18) | |

### EXAMPLE 3: Growing of cells

Cells were seeded as suspension cells, at a density of 10,000 cells/well in DMEM/F12 supplemented with 10 nM FTY720 or without FTY720 (control cells). The adherent cells were seeded at a density of 30,000 cells /well on poly *D*-lysine in DMEM/F12 supplemented with 1% fetal calf serum (FCS). When the cells had adhered (after 4 hours), the medium was changed to serum-free medium, and 10 nM FTY720, was added.

### EXAMPLE 4: Intracellular ATP - proliferation assay

Intracellular ATP levels have previously been shown to correlate to cell number (Crouch, Kozlowski et al. 1993). The following experiment was performed in sets of four parallel experiments (i.e., performed in quadruplicate) so that the cells could be used for different assays. FTY720 was added and cells were incubated at 37°C for 3 days. Cells were lysed with 0.1% Triton-X100 in Tris-EDTA buffer. Intracellular ATP was measured using an ATP-SL kit according to the manufacturer's instructions (BioThema, Sweden). Intracellular ATP was shown to correlate with cell number (Crouch, S. P., Kozlowski, R., 1993). For each experiment, wells were visually examined for signs of neurogenesis and counted to confirm the results of the assay. Results were repeatable and statistically significant.

### EXAMPLE 5: BrdU incorporation - proliferation assay

DNA synthesis is commonly used to measure cell proliferation. For such measurements, ³H-thymidine is traditionally used to label the DNA of mitotically active cells. In this experiment, ³H-thymidine was replaced by 5-bromo-2-deoxyuridine (BrdU). After incorporation if the pyrimidine analogue into DNA, BrdU was detected by immunoassay. The ELISA kit was provided by Roche, Germany.

### EXAMPLE 6: Lactate dehydrogenase (LDH) assay

The death of cells that occurred during the 3 days was measured as leakage of lactate dehydrogenase (LDH) into the medium. Viable cells do not leak LDH; only dead cells with damaged cell membranes leak LDH. The proportion of LDH in the medium to total LDH (medium + cells) gives the percentage of cell death. Treated and untreated cells can thus be compared to rule out apoptosis as the cause for the different results observed in the ATP proliferation assay. The amount of LDH was measured according to the instructions of the manufacturer, Promega, USA (see, also, Chen, et al., 2004).

### EXAMPLE 7: In situ hybridization

Sections (14 µm) of whole adult mouse brain were cut on a cryostat at -17°C, thawed onto microscope slides (Superfrost Plus; BDH, UK) and fixed in 4% formaldehyde for 5 min. Samples were deproteinated for 15 min in 0.2 M HCl, treated in 0.25% acetic anhydride in 0.1 M triethanolamine buffer (pH 8.0) for 20 min, and dehydrated in an ascending series of ethanol concentrations including a 5-min chloroform step before hybridization. To detect mouse S1PR mRNAs, antisense cRNA probes specific for S1P₁, S1P₅ were transcribed from a plasmid (pGEM-Teasy) containing the corresponding ORF cDNAs, which concurrently were labeled with [α-³⁵S] UTP (see Table 4).

**Table 4: S1PR cDNA probes for in-situ hybridization**

| Gene | Accession No | Size of cDNA probe (bp) |
|---|---|---|
| S1P₁ | NM_007901 | 172 (Position 1695-1866) |
| S1P₅ | NM_053190 | 294 (Position 55-347) |

Sections were incubated with the probe at 55°C for 16 hr in a hybridization buffer containing 52% formamide, 10% dextran sulfate, 208 mM NaCl, 2% 50 x Denhardt's solution (1% Ficoll, 1% polyvinylpyrrolidone, 1% bovine serum albumin (BSA)), 10 mM Tris pH 8.0,1 mM EDTA, 500 ng/ml yeast tRNA, 10 mM dithiothreitol (DTT) and 20 x 10⁶ cpm probe per ml buffer. After hybridization, the sections were treated with RNase A, 10 µg/ml in 0.5 M NaCl at 37°C for 30 min. Samples were washed in 4 x saline sodium citrate (SSC; 1 x SSC is 0.15 M sodium chloride and 0.015 M trisodium citrate, pH 7.0) for 20 min, 2 x SSC for 10 min, 1 x SSC for 10 min, and 0.5 x SSC for 10 min at room temperature. A high stringency wash was carried out at 70°C for 30 min in 0.1 x SSC. All wash steps included the addition of 1 mM DTT.

The sections were dehydrated in an ascending series of ethanol concentrations, dried overnight, and mounted in cassettes with autoradiographic films (Beta-max, Amersham) for 3 weeks. The films were developed in Kodak D-19 developer, fixed in Kodak RA-3000 diluted 1:3, rinsed, and dried. Sections were then dipped in Kodak NTB-2 nuclear track emulsion diluted 1:1, exposed for 6 weeks, developed in Kodak D-19 for 3 min, fixed in Kodak RA-3000 fixer, and counterstained with cresyl violet. The specificity of the hybridization was tested using a sense probe transcribed from the same plasmids. No hybridization signal was obtained under this condition. The emulsion-dipped sections were analyzed using a Nikon E600 microscope.

### EXAMPLE 8: In vivo proliferation experiment

For these studies, 11.6 mg of FTY720 was dissolved in phosphate buffered saline (PBS) containing 0.1% mouse serum to a concentration of 0.5 mg/ml. The solution was further diluted to 0.25 mg/ml in PBS plus 0.1% mouse serum. BrdU was added to a final concentration of 6.25 mg/ml. Adult (>8 weeks) male C57BL6 mice received one 200 µl IP injection every 24 h for seven days and were then sacrificed with CO₂. One cohort of animals was allowed to live for an additional 14 days before sacrificing. Control injections consisted of BrdU at the same concentration in PBS plus 0.1% mouse serum. The brains were dissected out and snap frozen. The FTY720 solution at 0.5 mg/ml showed precipitates after storage in 4° or -20°. After further dilution to 0.25mg/ml, vortexing and heating to 37° the amount of precipitates was significantly reduced.

### EXAMPLE 9: Results for expression analysis

These studies have investigated the mRNA and protein expression pattern of FTY720P-responsive receptors in the adult mouse brain. The results indicated that S1P₁ and S1P₅ are expressed in neurogenic regions of adult mouse brain. Using RT-PCR, it was found that all S1PR mRNAs are expressed in either the lateral ventricle wall tissue or in neurospheres derived from cultured neural stems cells (NSCs) derived from this tissue (Table 5).

**Table 5: Expression of S1PR mRNA in adult mouse brain**

| **RT-PCR** | **S1P₁** | **S1P₂** | **S1P₃** | **S1P₄** | **S1P₅** |
|---|---|---|---|---|---|
| Neurospheres | +++ | + | +++ | + | - |
| Lateral ventricle wall | +++ | - + +++ | + | +++ | + |
| Rest of brain | +++ | - | ++ | ++ | ++ |

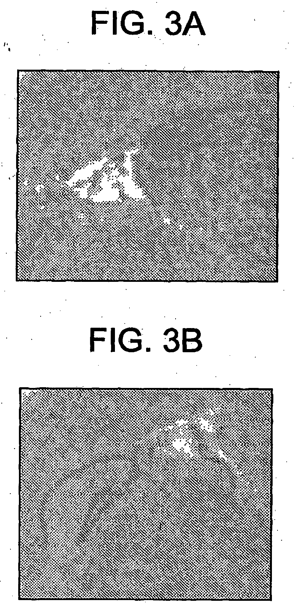

Table 5. Expression levels estimated by RT-PCR (on a 1-3 scale). *In situ* hybridization results are shown in FIG. 3.

The *In situ* hybridization technique was also used to investigate the brain areas in which the S1PR receptor proteins are expressed. Previous work performed by others using this technique on rat embryonic brain revealed that S1P₁ is primarily expressed in the subventricular zone (SVZ) of the lateral ventricle wall (LVW). In contrast, S1P₃ is mostly scattered in a punctate pattern and co localized with vascular endothelial markers, which indicates a role in angiogenesis (McGiffert, et al., 2002). The data shown herein indicate that S1P1 expression in the adult mouse brain is confined to the SVZ of the LVW, expanding into the rostral migratory stream. The S1P₅ receptor is expressed in the choroid plexus, hippocampus (dentate gyrus and CA1-CA3), and piriform cortex, which is another area of adult neurogenesis. No hybridization was observed for S1P₂ or S1P₃ in the adult mouse brain. Notably, the S1P₁ and S1P₅ receptors have been found to binding FTY720P with the highest affinity (Table 3). These two receptors are therefore the most likely targets for FTY720 inducement of proliferation as demonstrated herein.

### EXAMPLE 10: Results for in vitro proliferation assays

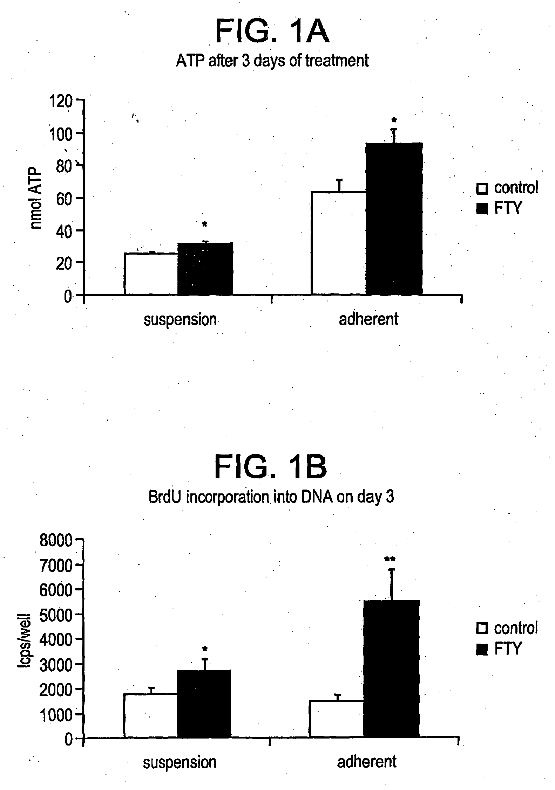

It was determined that FTY720P induces *in vitro* proliferation of adult neural stem cells. Using the ATP assay, increases of 25% and 42% in intracellular ATP levels (and hence cell numbers) was seen in FTY720-treated suspension and adherent cells, respectively. To confirm proliferation, incorporation of BrdU was used to assess DNA synthesis. Increases of 52% and 271% in BrdU incorporation were measured in FTY720-treated suspension and adherent cells, respectively. The increases in ATP and BrdU incorporation were determined to be statistically significant (FIG. 1). In a separate experiment, a dose-response curve was performed on NSCs, which revealed a very low EC₅₀ for FTY720: 0.02 nM (FIG. 2). The EC₅₀ value for FTY720 is in the same range as the EC₅₀ value for EGF, indicating that FTY720 is an extremely potent mitogen for NSCs. To ensure that the differences in cell number were not the result of differences in apoptosis levels, LDH levels (an assay measuring cell death) were measured. No significant change in LDH levels between control and FTY720-treated cells was observed.

### EXAMPLE 11: In vivo experiments to characterize FTY720

To characterize FTY720-stimulation of neurogenesis, *in vivo* studies can be performed. Such studies can be modeled on the intraventricular infusion experiments used to test the impact of growth factors on neurogenesis. Infusion of both EGF and basic FGF have been shown to proliferate the ventricle wall cell population, and in the case of EGF, extensive migration of progenitors into the neighboring striatal parenchyma (Craig, C. G., V. Tropepe, et al. 1996; Kuhn, H. G., J. Winkler, et al. 1997). Differentiation of the progenitors was predominantly into a glial lineage while reducing the generation of neurons (Kuhn, H. G., J. Winkler, et al. 1997). A recent study found that intraventricular infusion of BDNF in adult rats promotes increases the number of newly generated neurons in the olfactory bulb and rostral migratory stream, and in parenchymal structures, including the striatum, septum, thalamus and hypothalamus (Pencea, V., K. D. Bingaman, et al. 2001).

To determine the effects of FTY720 on neurogenesis, the compound can be administered systemically or topically (for example, intranasally, orally, intraperitoneally, or intravenously) at a range of concentrations into mice and/or rats. The basic experimental set up for infusion of compounds into the rodent lateral ventricle and the detection of new neurons and glia is described below.

Evidence of a role for FTY720 activity through the S1P receptors can be gained by the use of knockout mice for these molecules, either singly or in combination. The expression pattern for S1P₁ and S1P₅ in the adult mouse brain and the high affinity for FTY720P compared to other S1PRs (Table 3), makes S1P₁ and /or S1P₅ likely targets for FTY720 in neural stem cells, consistent with the data shown herein. Experimentation using S1P receptor knockout mice, with or without intraventicular infusion of FTY720 will assist in deciphering the precise role of each receptor in neurogenesis. These studies can be used to determine the effects of FTY720 functioning through one or more of the EDG receptor family members.

### EXAMPLE 12: Clinical applications for FTY720

One goal is to characterize the ability of FTY720 to proliferate NSCs through the stimulation of the S1PRs (e.g., S1P₁ or S1P₅). FTY720-induced stimulation of neural stem cell activity will be beneficial in alleviating symptoms of a number of disorders of the nervous system, e.g. Parkinson's disease, Alzheimer's disease, all forms of depression, cognitive impairment, schizophrenia, Huntington's disease, and trauma such as spinal cord injury. Besides inducing neural stem cell activity, FTY720s' anti-inflammatory activity could also act in a synergistic manner to treat Parkinson's.

A recent study has highlighted the possibility of an alternative approach to the delivery of compounds to the ventricular system by nasal application or "sniffing" (Born, J., T. Lange, et al. 2002). This means of delivery, similarly to intraventricular infusion, essentially bypasses systemic side effects of the applied compound. Successful results from the above experiments will be carried out to assess this application approach. To address various diseases, FTY720 compositions may be characterized in rodent and non-human primate disease models as treatments.

### Animal models

FTY720 will be characterized in the following animal models of CNS disease/disorders/trauma to demonstrate recovery. Exemplary models are listed below; additional/modified models will also be used:
**Models of epilepsia** such as electroshock-induced seizures (Billington A et al., Neuroreport 2000 Nov 27;11(17):3817-22), pentylene tetrazol (Gamaniel K et al., Prostaglandins Leukot Essent Fatty Acids 1989 Feb;35(2):63-8) or kainic acid (Riban V et al, Neuroscience 2002;112(1):101-11) induced seizures;
**Models of psychosis/schizophrenia** such as amphetamine-induced stereotypies/locomotion (Borison RL & Diamond BI, Biol Psychiatry 1978 Apr;13(2):217-25), MK-801 induced stereotypies (Tiedtke et al., J Neural Transm Gen Sect 1990;81(3):173-82), MAM (methyl azoxy methanol-induced (Fiore M et al., Neuropharmacology 1999 Jun;38(6):857-69; Talamini LM et al., Brain Res 1999 Nov 13;847(1):105-20) or reeler model (Ballmaier M et al., Eur J Neurosci 2002 Apr;15(17): 1197-205);
**Models of Parkinson's disease** such as MPTP (Schmidt &Ferger, J Neural Transm 2001;108(11):1263-82), 6-OH dopamine (O'Dell & Marshall, Neuroreport 1996 Nov 4;7(15-17):2457-61) induced degeneration;
**Models of Alzheimer's disease** such as fimbria fornix lesion model (Krugel et al., Int J Dev Neurosci 2001 Jun;19(3):263-77), basal forebrain lesion model (Moyse E et al., Brain Res 1993 Apr 2;607(1-2):154-60);
**Models of stroke** such as focal ischemia (Schwartz DA et al., Brain Res Mol Brain Res 2002 May 30;101(1-2):12-22); global ischemia (2- or 4-vessel occlusion) (Roof RL et al., Stroke 2001 Nov;32(11):2648-57; Yagita Y et al., Stroke 2001 Aug;32(8): 1890-6);
**Models of amyotrophic lateral sclerosis** such as pmn mouse model (Kennel P et al., J Neurol Sci 2000 Nov 1;180(1-2):55-61);
**Models of anxiety** such as elevated plus-maze test (Holmes A et al., Behav Neurosci 2001 Oct;115(5):1129-44), marble burying test (Broekkamp et al., Eur J Pharmacol 1986 Jul 31;126(3):223-9), open field test (Pelleymounter et al., J Pharmacol Exp Ther 2002 Jul;302(1):145-52);
**Models of depression** such as learned helplessness test, forced swim test (Shirayama Y et al., J Neurosci 2002 Apr 15;22(8):3251-61), bulbectomy (O'Connor et al., Prog Neuropsychopharmacol Biol Psychiatry 1988;12(1):41-51);
**Models for learning/memory** such as Morris water maze test (Schenk F & Morris RG, Exp Brain Res 1985;58(1):11-28);
**Models for Huntington's disease** such as quinolinic acid injection (Marco S et al., J Neurobiol 2002 Mar;50(4):323-32), transgenics/knock-ins (reviewed in Menalled LB and Chesselet MF, Trends Pharmacol Sci. 2002 Jan;23(1):32-9); and
**Models for aging** using old mice/rats.

These models are contemplated with any particular adaptations needed for the method to be compliant with the FTY720 composition administered and delivery system including formulation of the composition intended.

The investigation of the role of relevant ligands/receptors *in vivo* using healthy and/or models for disease/trauma/disorders will be conducted according to the following protocol (intracerebroventricular administration), here described for rats, but available also for mince:

### Neurogenesis - In vivo testing of compounds:

Animals: Male rats (a corresponding protocol for mice will also be used). Animal housing: 12 hours light /dark regime; feeding: standard pellets; feeding and drinking ad libitum; 5 animals in standard cage;
Compound administration: Brain infusion by osmotic mini-pumps for 1-14 days of BrdU or ³H-thymidine or other marker of proliferation, and relevant compound. Survival for 0-4 weeks post infusion.
**Operation:** Animal handling and surgery as in Pencea V et al., (2001).
**Removal of pumps:** 1-14 days after insertion of pump: anesthesia of animals.
**Brain Sample Collection:** Narcosis of animals; transcardial perfusion with NaCl; perfusion with paraformaldehyde (4%) solution; removal of brain store in paraformaldehyde (4%) solution over night; transfer in 30% sucrose solution at 4°C; separating bulbus olfactorius (OB); freezing at -80°C in methylbutan and storage in - 80°C freezer.
**Sectioning:** Sagittal sectioning of ipsilateral OB and coronal sectioning of rest of brain on cryotom.
**Immunohistochemistry:** Analysis and quantification will be done for proliferative brain regions, migratory streams, and areas of clinical relevance (some, but not all, of these areas are exemplified below).

DAB (diamine benzidine) or fluorescence visualization using one or several of the following antibodies: as neuronal markers NeuN, Tuj1, anti-tyrosine hydroxylase, anti-MAP-2 etc.; as glial markers anti-GFAP, anti-S100 etc.; as oligodendrocyte markers anti-GalC, anti-PLP etc. For BrdU visualization: anti-BrdU.
**Quantification:** I) DAB-BrdU=Immunohistochemistry and stereological quantification in ipsilateral brain regions. II) 4-weeks-survival-group: ipsilateral hemisphere; a) Quantification of BrdU positive cells via DAB-Immunohistochemistry (stereology) for dorsal hippocampus dentate gyrus, dorsal hippocampus CA1/alveus, olfactory bulb (OB), subventricular zone (SVZ), and striatum; b) Quantification of double-staining with confocal microscope for every (OB, DG, CA1/alveus, SVZ, wall-to-striatum) structure: checking of BrdU+ for double-staining with the lineage markers. Further experimental details can be found in Pencea V et al., J. Neurosci Sept 1 (2001), 21(17):6706-17.
**Differentiation analysis:** Quantitative Polymerase Chain Reaction (QPCR) or Laser Scanning Cytometry (LSC) can be performed. Microarray analysis and proteomic-based studies using SELDI (surface-enhanced laser desorption/ionization) mass spectroscopy can also be used.

The details of one or more embodiments of the invention have been set forth in the accompanying description above. Although any methods and materials similar or equivalent to those described herein can be used in the practice or testing of the present invention, the preferred methods and materials are now described. Other features, objects, and advantages of the invention will be apparent from the description and from the claims.

In the specification and the appended claims, the singular forms include plural referents unless the context clearly dictates otherwise. Unless defined otherwise, all technical and scientific terms used herein have the same meaning as commonly understood by one of ordinary skill in the art to which this invention belongs. Unless expressly stated otherwise, the techniques employed or contemplated herein are standard methodologies well known to one of ordinary skill in the art.

### References

Adachi, K. et al., Bioorganic and Medicinal Chemistry Letters (1995), 5:8 853-856.
Altman, J. and G. Das (1965). "Autoradiographic and histological evidence of postnatal hippocampal neurogenesis in rats." J Comp Neurol 124: 319-335.
Altman, J. and G. Dash (1967). "Postnatal neurogenesis in the guinea-pig." Nature 214: 1098-1101.
Anliker, B. & Chun, J. (2004) Lysophospholipid G protein-coupled receptors, J Biol Chem. 279, 20555-8.
Beer, M. S., Stanton, J. A., Salim, K., Rigby, M., Heavens, R. P., Smith, D. & McAllister, G. (2000) EDG receptors as a therapeutic target in the nervous system, Ann N Y Acad Sci. 905, 118-31.
Biebl, M., C. M. Cooper, et al. (2000). "Analysis of neurogenesis and programmed cell death reveals a self- renewing capacity in the adult rat brain." Neurosci Lett 291(1): 17-20.
Bjorklund, A. and O. Lindvall (2000). "Cell replacement therapies for central nervous system disorders. " Nat Neurosci 3(6): 537-44.
Born, J., T. Lange, et al. (2002) Nat Neurosci 5(6): 514-6.
Brinkmann et al, Transplantation. (2001) Sep 15;72(5):764-9.
Brinkmann, V., Davis, M. D., Heise, C. E., Albert, R., Cottens, S., Hof, R., Bruns, C., Prieschl, E., Baumruker, T., Hiestand, P., Foster, C. A., Zollinger, M. & Lynch, K. R. (2002) The immune modulator FTY720 targets sphingosine 1-phosphate receptors, J Biol Chem. 277, 21453-7.
Chae, S. S., Proia, R. L. & Hla, T. (2004) Constitutive expression of the S1P1 receptor in adult tissues, Prostaglandins Other Lipid Mediat. 73, 141-50.
Chiba, K., Yanagawa, Y., Kataoka, H., Kawaguchi, T., Ohtsuki, M. & Hoshino, Y. (1999) FTY720, a novel immunosuppressant, induces sequestration of circulating lymphocytes by acceleration of lymphocyte homing, Transplant Proc. 31, 1230-3.
Chun, J. (1999) Lysophospholipid receptors: implications for neural signaling, Crit Rev Neurobiol. 13,151-68.
Colthorpe P, Farr SJ, Taylor G, Smith IJ, Wyatt D. (1992) The pharmacokinetics of pulmonary-delivered insulin: a comparison of intratracheal and aerosol administration to the rabbit. Pharm Res. Jun;9(6):764-8.
Contos, J. J., N. Fukushima, et al. (2000). "Requirement for the 1pA1 lysophosphatidic acid receptor gene in normal suckling behavior." Proc Natl Acad Sci U S A 97(24): 13384-9.
Craig, C. G., V. Tropepe, et al. (1996). "In vivo growth factor expansion of endogenous subependymal neural precursor cell populations in the adult mouse brain." J Neurosci 16(8): 2649-58.
Crouch, S. P., Kozlowski, R., Slater, K. J. & Fletcher, J. (1993) The use of ATP bioluminescence as a measure of cell proliferation and cytotoxicity, J Immunol Methods. 160, 81-8.
Chen, S. L., Chen, T. M. & Wang, H. J. (2004) Free thoracodorsal artery perforator flap in extremity reconstruction: 12 cases, Br J Plast Surg. 57, 525-30.
Czlonkowska, A., Kurkowska-Jastrzebska, I., Czlonkowski, A., Peter, D. & Stefano, G. B. (2002) Immune processes in the pathogenesis of Parkinson's disease - a potential role for microglia and nitric oxide, Med Sci Monit. 8, RA165-77.
Doetsch, F., I. Caille, et al. (1999). "Subventricular zone astrocytes are neural stem cells in the adult mammalian brain." Cell 97(6): 703-16.
Ekdahl, C. T., Claasen, J. H., Bonde, S., Kokaia, Z. & Lindvall, O. (2003) Inflammation is detrimental for neurogenesis in adult brain, Proc Natl Acad Sci U S A. 100,13632-7.
Elliott, RB, Edgar, BW, Pilcher, CC, et al (1987) Parenteral absorption of insulin from the lung in diabetic children. Aust Paediatr J 23,293-297.
Fujino, M., Funeshima, N., Kitazawa, Y., Kimura, H., Amemiya, H., Suzuki, S. & Li, X. K. (2003) Amelioration of experimental autoimmune encephalomyelitis in Lewis rats by FTY720 treatment, J Pharmacol Exp Ther. 305, 70-7.
Fukushima, N., Ishii, I., Contos, J. J., Weiner, J. A. & Chun, J. (2001) Lysophospholipid receptors. Annu Rev Pharmacol Toxicol. 41, 507-34.
Gage, F. H., G. Kempermann, et al. (1998). "Multipotent progenitor cells in the adult dentate gyrus." J Neurobiol 36(2): 249-66.
Gansslen M. (1925) Über Inhalation von Insulin. Klin Wochenschr; 4:71.
Goetzl, E. J. & An, S. (1998) Diversity of cellular receptors and functions for the lysophospholipid growth factors lysophosphatidic acid and sphingosine 1-phosphate, FASEB J. 12,1589-98.
Govinda (1959) Indian J. Physiol. Pharmacol. 3:161-167.
Graler, M. H., Bernhardt, G. & Lipp, M. (1998) EDG6, a novel G-protein-coupled receptor related to receptors for bioactive lysophospholipids, is specifically expressed in lymphoid tissue, Genomics. 53, 164-9.
Glickman, M., Malek, R. L., Kwitek-Black, A. E., Jacob, H. J. & Lee, N. H. (1999) Molecular cloning, tissue-specific expression, and chromosomal localization of a novel nerve growth factor-regulated G-protein-coupled receptor, nrg-1, Mol Cell Neurosci. 14, 141-52.
Hale, J. J., Neway, W., Mills, S. G., Hajdu, R., Ann Keohane, C., Rosenbach, M., Milligan, J., Shei, G. J., Chrebet, G., Bergstrom, J., Card, D., Koo, G. C., Koprak, S. L., Jackson, J. J., Rosen, H. & Mandala, S. (2004) Potent S1P receptor agonists replicate the pharmacologic actions of the novel immune modulator FTY720, Bioorg Med Chem Lett. 14, 3351-5.
Harada, J. et al. (2001) Abstract 31st Annual Meeting of the Society for Neuroscience, San Diego, CA, November 10-15,2001.
Harada, J., Foley, M., Moskowitz, M. A. & Waeber, C. (2004) Sphingosine-1-phosphate induces proliferation and morphological changes of neural progenitor cells, J Neurochem. 88,1026-39.
Hastings RH, Grady M, Sakuma T, et al. (1992) Clearance of different-sized protein from the alveolar space in humans and rabbits. J Appl Physiol 73:1310-1316.
Herman, J. P. and N. D. Abrous (1994). "Dopaminergic neural grafts after fifteen years: results and perspectives." Prog Neurobiol 44(1): 1-35.
Hisanaga, K., Asagi, M., Itoyama, Y. & Iwasaki, Y. (2001) Increase in peripheral CD4 bright+ CD8 dull+ T cells in Parkinson disease, Arch Neurol. 58, 1580-3.
Im, D. S., Clemens, J., Macdonald, T. L. & Lynch, K. R. (2001) Characterization of the human and mouse sphingosine 1-phosphate receptor, S1P5 (Edg-8): structure-activity relationship of sphingosine1-phosphate receptors, Biochemistry. 40, 14053-60.
Im, D. S., Heise, C. E., Ancellin, N., O'Dowd, B. F., Shei, G. J., Heavens, R. P., Rigby, M. R., Hla, T., Mandala, S., McAllister, G., George, S. R. & Lynch, K. R. (2000) Characterization of a novel sphingosine 1-phosphate receptor, Edg-8, J Biol Chem. 275, 14281-6.
Jacobson, M. (1991). Histosenesis and morphogenesis of cortical structures. Developmental Neurobiology, Plenum Press, New York: 401-451.
Johansson, C. B., S. Momma, et al. (1999). "Identification of a neural stem cell in the adult mammalian central nervous system." Cell 96(1): 25-34.
Johansson, C. B., M. Svensson, et al. (1999). "Neural stem cells in the adult human brain." Exp Cell Res 253(2): 733-6.
Johe, K. K., T. G. Hazel, et al. (1996). "Single factors direct the differentiation of stem cells from the fetal and adult central nervous system." Genes Dev 10(24): 3129-40.
Kohler, D, Schluter, KJ, Kerp, L, et al (1987) Nicht radioactives verfahren zur messung der lungenpermeabilitat: inhalation von insulin. Atemw Lungenkrkh 13,230-232.
Kuhn, H. G. and C. N. Svendsen (1999). "Origins, functions, and potential of adult neural stem cells." Bioessays 21(8): 625-30.
Kuhn, H. G., J. Winkler, et al. (1997). "Epidermal growth factor and fibroblast growth factor-2 have different effects on neural progenitors in the adult rat brain." J Neurosci 17(15): 5820-9.
Laube BL. (1993) Preliminary study of insulin aerosol delivered by oral inhalation in diabetic patients. JAMA 269:2106-9.
Lee et al. (2004) FTY720 induces apoptosis of human hepatoma cell lines through PI3-K-mediated Akt dephosphorylation. Carcinogenesis, Aug 5 Epub ahead of print.
Lee and Sciara (1976) J. Pharm. Sci. 65:567-572.
Liu, Y., Wada, R., Yamashita, T., Mi, Y., Deng, C. X., Hobson, J. P., Rosenfeldt, H. M., Nava, V. E., Chae, S. S., Lee, M. J., Liu, C. H., Hla, T., Spiegel, S. & Proia, R. L. (2000) Edg-1, the G protein-coupled receptor for sphingosine-1-phosphate, is essential for vascular maturation, J Clin Invest. 106, 951-61.
Lois, C. and A. Alvarez-Buylla (1993). "Proliferating subventricular zone cells in the adult mammalian forebrain can differentiate into neurons and glia." Proc Natl Acad Sci U S A 90(5): 2074-7.
Mandala et al.(2002) Science 296:346.
Magavi, S. S., B. R. Leavitt, et al. (2000). "Induction of neurogenesis in the neocortex of adult mice [see comments]." Nature 405(6789): 951-5.
Maki, T., Gottschalk, R. & Monaco, A. P. (2002) Prevention of autoimmune diabetes by FTY720 in nonobese diabetic mice, Transplantation. 74,1684-6.
Mandala, S., Hajdu, R., Bergstrom, J., Quackenbush, E., Xie, J., Milligan, J., Thornton, R., Shei, G. J., Card, D., Keohane, C., Rosenbach, M., Hale, J., Lynch, C. L., Rupprecht, K., Parsons, W. & Rosen, H. (2002) Alteration of lymphocyte trafficking by sphingosine-1-phosphate receptor agonists, Science. 296, 346-9.
Masubuchi, Y., Kawaguchi, T., Ohtsuki, M., Suzuki, C., Amano, Y., Hoshino, Y. & Chiba, K. (1996) FTY720, a novel immunosuppressant, possessing unique mechanisms. IV. Prevention of graft versus host reactions in rats, Transplant Proc. 28, 1064-5.
Matsuura, M., Imayoshi, T. & Okumoto, T. (2000) Effect of FTY720, a novel immunosuppressant, on adjuvant- and collagen-induced arthritis in rats, Int J Immunopharmacol. 22, 323-31.
Matsuura, M., Imayoshi, T., Chiba, K. & Okumoto, T. (2000) Effect of FTY720, a novel immunosuppressant, on adjuvant-induced arthritis in rats, Inflamm Res. 49, 404-10.
McGiffert, C., Contos, J. J., Friedman, B. & Chun, J. (2002) Embryonic brain expression analysis of lysophospholipid receptor genes suggests roles for s1p(1) in neurogenesis and s1p(1-3) in angiogenesis, FEBS Lett. 531, 103-8.
McKay, R. (1997). "Stem cells in the central nervous system." Science 276(5309): 66-71.
Momma, S., C. B. Johansson, et al. (2000). "Get to know your stem cells." Curr Opin Neurobiol 10(1): 45-9.
Nagai et al. (1984) J. Contr. Rel. 1:15-22.
Nagano et al. (1985) Jikeikai Med. J. 32:503-506.
Palmer, T. D., E. A. Markakis, et al. (1999). "Fibroblast growth factor-2 activates a latent neurogenic program in neural stem cells from diverse regions of the adult CNS." J Neurosci 19(19): 8487-97.
Pencea, V., K. D. Bingaman, et al. (2001). "Infusion of Brain-Derived Neurotrophic Factor into the Lateral Ventricle of the Adult Rat Leads to New Neurons in the Parenchyma of the Striatum, Septum, Thalamus, and Hypothalamus." J Neurosci 21(17): 6706-17.
Radeff-Huang, J., Seasholtz, T. M., Matteo, R. G. & Brown, J. H. (2004) G protein mediated signaling pathways in lysophospholipid induced cell proliferation and survival, J Cell Biochem. 92, 949-66.
Rajan, P. and R. D. McKay (1998). "Multiple routes to astrocytic differentiation in the CNS." J Neurosci 18(10): 3620-9.
Sanchez, T. & Hla, T. (2004) Structural and functional characteristics of S1P receptors, J Cell Biochem. 92, 913-22.
Sakr (1992) Int. J. Phar. 86:1-7.
Schlutiter et al. (Abstract) (1984) Diabetes 33:75A.
Shinomiya, T., Li, X. K., Amemiya, H. & Suzuki, S. (1997) An immunosuppressive agent, FTY720, increases intracellular concentration of calcium ion and induces apoptosis in HL-60, Immunology. 91,594-600.
Snyder, E. Y., C. Yoon, et al. (1997) "Multipotent neural precursors can differentiate toward replacement of neurons undergoing targeted apoptotic degeneration in adult mouse neocortex." Proc Natl Acad Sci USA 94(21): 11663-8.
Tedesco-Silva H, et al. (2004) FTY720, a novel immunomodulator: efficacy and safety results from the first phase 2A study in de novo renal transplantation. Transplantation. Jun 27;77(12):1826-33.
Toman, R. E. & Spiegel, S. (2002) Lysophospholipid receptors in the nervous system, Neurochem Res. 27, 619-27.
Usui, S., Sugimoto, N., Takuwa, N., Sakagami, S., Takata, S., Kaneko, S. & Takuwa, Y. (2004) Blood lipid mediator sphirigosine 1-phosphate potently stimulates platelet-derived growth factor-A and -B chain expression through S1P1-Gi-Ras-MAPK-dependent induction of Kruppel-like factor 5, J Biol Chem. 279, 12300-11.
Webb, M., Tham, C. S., Lin, F. F., Lariosa-Willingham, K., Yu, N., Hale, J., Mandala, S., Chun, J. & Rao, T. S. (2004) Sphingosine 1-phosphate receptor agonists attenuate relapsing-remitting experimental autoimmune encephalitis in SJL mice, J Neuroimmunol. 153, 108-21.
Williams, B. P., J. K. Park, et al. (1997). "A PDGF-regulated immediate early gene response initiates neuronal differentiation in ventricular zone progenitor cells." Neuron 18(4): 553-62.
Yang, Z., Chen, M., Fialkow, L. B., Ellett, J. D., Wu, R, Brinkmann, V., Nadler, J. L. & Lynch, K. R. (2003) The immune modulator FYT720 prevents autoimmune diabetes in nonobese diabetic mice small star, filled, Clin Immunol. 107, 30-5.
Yamazaki, Y., J. Kon, et al. (2000). "Edg-6 as a putative sphingosine 1-phosphate receptor coupling to Ca(2+) signaling pathway." Biochem Biophys Res Commun 268(2): 583-9.
Yoshida et al. (1987) Clin. Res. 35:160-166.
Zhang, G., Contos, J. J., Weiner, J. A., Fukushima, N. & Chun, J. (1999) Comparative analysis of three murine G-protein coupled receptors activated by sphingosine-1-phosphate, Gene. 227, 89-99.

All patents and publications cited in this specification are hereby incorporated by reference herein, including the previous disclosure provided by U.S. application 60/502,386 filed September 12, 2003, U.S. Patent Application 10/434,943 filed May 8, 2003, U.S. Patent Application 60/393,159 filed July 2, 2002, and U.S. Patent Application 60/379,114 filed May 8, 2002.

## Claims

1. Use of FTY720 or a derivative thereof selected from R-AAL, phosphate ester metabolites of FTY720, pharmaceutically acceptable salts of FTY720, or pegylated FTY720 in the manufacture of a composition for use in treating a neurodegenerative disease, a neurological disease or a psychiatric disease, with the proviso that the disease is not multiple sclerosis (MS) or a demyelinating disease such as Guillain-Barre syndrome.

2. The use of claim 1, wherein said neurodegenerative disease, neurological disease or a psychiatric disease is selected from neurodegenerative disorders, neural stem cell disorders, neural progenitor disorders, ischemic disorders, affective disorders, neuropsychiatric disorders, and learning and memory disorders.

3. The use of claim 1, wherein said neurodegenerative disease, neurological disease or a psychiatric disease is selected from Parkinson's disease, Parkinsonian disorders, Huntington's disease, Alzheimer's disease, amyotrophic lateral sclerosis, spinal ischemia, ischemic stroke, spinal cord injury, cancer-related brain injury, and cancer-related spinal cord injury, Shy-Drager syndrome, progressive supranuclear palsy, Lewy body disease, stroke, cerebral infarction, multi-infarct dementia, geriatric dementia, and depression.

4. The user of claim 1 wherein the neurodegenerative disease is Parkinson's disease or Alzheimer's disease.

5. The use of claim 1 wherein the psychiatric disease is depression.

6. The use of claim 1, wherein said derivative is FTY720P.

7. The use of claim 1, wherein said FTY720 or derivative is for administration at a concentration of 1 ng/kg/day to 1 mg/kg/day, preferably 1 µg/kg/day to 0.1 mg/kg/day, more preferably 5 µg/kg to approximately 0.07 mg/kg.

8. The use of claim 1, wherein said FTY720 or derivative is for administration in an amount of 0.3 mg to 10 mg.

9. The use of claim 1, wherein said FTY720 or derivative is for administration to a mammal.

10. The use of claim 9, wherein said FTY720 or derivative is for administration to a human.

11. The use of claim 10, wherein said FTY720 or derivative is for administration to an adult.

12. The use of claim 1, wherein said FTY720 or derivative is administered in combination with one or more growth factors.

13. The use of claim 1, wherein said FTY720 or derivative is administered in combination with one or more agents selected from anti-depressants, anti-anxiety treatments, anti-psychotic treatments, epilepsy treatments, Alzheimer's treatments, Parkinson's treatments, MAO inhibitors, serotonin-uptake blockers, noradrenaline uptake blockers, dopamine uptake blockers, dopamine agonists, L-DOPA, tranquilizers, sedatives and lithium.

14. The use of claim 1, wherein said FTY720 or derivative is for administration systemically.

15. The use of claim 1, wherein said composition is for oral, subcutaneous, intraperitoneal, intramuscular, intraventricular, intraparenchymal, intrathecal, intracranial, buccal, mucosal, nasal or rectal administration.

16. The use of claim 1, wherein said composition is formulated as a nasal spray or nasal suppository.

17. The use of claim 16, wherein said composition is for administration by a dry powder inhaler or aqueous-based inhaler.

18. The use of claim 1, wherein said FTY720 or a derivative thereof is for administration to the central nervous system of the subject.

19. The use of any claims 1 to 18, wherein the phosphate ester of FTY720 is selected from FTY720P and R-AFD.

20. The use of any claims 1 to 18, wherein said derivative of FTY720 is selected from FTY720P, R-AAL, and R-AFD.

## Patentansprüche

1. Verwendung von FTY720 oder eines Derivats davon, ausgewählt aus R-AAL, Phosphatester-Metaboliten von FTY720, pharmazeutisch annehmbaren Salzen von FTY720 oder PEGyliertem FTY720 bei der Herstellung einer Zusammensetzung zur Verwendung beim Behandeln einer neurodegenerativen Krankheit, einer neurologischen Krankheit oder einer psychiatrischen Krankheit mit der Maßgabe, dass es sich bei der Krankheit nicht um multiple Sklerose (MS) oder um eine demyelinisierende Krankheit wie zum Beispiel das Guillain-Barré-Syndrom handelt.

2. Verwendung nach Anspruch 1, wobei die besagte neurodegenerative Krankheit, neurologische Krankheit oder psychiatrische Krankheit aus neurodegenerativen Funktionsstörungen, Funktionsstörungen von neuralen Stammzellen, Funktionsstörungen von neuralen Progenitoren, ischämischen Funktionsstörungen, affektiven Störungen, neuropsychiatrischen Störungen und Lern- und Gedächtnisstörungen ausgewählt ist.

3. Verwendung nach Anspruch 1, wobei die besagte neurodegenerative Krankheit, neurologische Krankheit oder psychiatrische Krankheit aus Morbus Parkinson, parkinsonoiden Funktionsstörungen, Huntington-Krankheit, Morbus Alzheimer, amyotropher Lateralsklerose, spinaler Ischämie, ischämischem Schlaganfall, Rückenmarksschädigung, mit Krebs verbundener Hirnschädigung und mit Krebs verbundener Rückenmarksschädigung, Shy-Drager-Syndrom, progressiver supranukleärer Blickparese, Lewy-Körper-Krankheit, Schlaganfall, zerebralem Infarkt, Multiinfarktdemenz, Altersdemenz und Depression ausgewählt ist.

4. Verwendung nach Anspruch 1, wobei es sich bei der neurodegenerativen Krankheit um Morbus Parkinson oder um Morbus Alzheimer handelt.

5. Verwendung nach Anspruch 1, wobei es sich bei der psychiatrischen Krankheit um Depression handelt.

6. Verwendung nach Anspruch 1, wobei es sich bei dem besagten Derivat um FTY720P handelt.

7. Verwendung nach Anspruch 1, wobei das besagte FTY720 oder Derivat zur Verabreichung in einer Konzentration von 1 ng/kg/Tag bis 1 mg/kg/Tag, vorzugsweise 1 µg/kg/Tag bis 0,1 mg/kg/Tag, bevorzugter 5 µg/kg bis ungefähr 0,07 mg/kg vorgesehen ist.

8. Verwendung nach Anspruch 1, wobei das besagte FTY720 oder Derivat zur Verabreichung in einer Menge von 0,3 mg bis 10 mg vorgesehen ist.

9. Verwendung nach Anspruch 1, wobei das besagte FTY720 oder Derivat zur Verabreichung an einen Säuger vorgesehen ist.

10. Verwendung nach Anspruch 9, wobei das besagte FTY720 oder Derivat zur Verabreichung an einen Menschen vorgesehen ist.

11. Verwendung nach Anspruch 10, wobei das besagte FTY720 oder Derivat zur Verabreichung an einen Erwachsenen vorgesehen ist.

12. Verwendung nach Anspruch 1, wobei das besagte FTY720 oder Derivat in Kombination mit einem oder mehreren Wachstumsfaktoren verabreicht wird.

13. Verwendung nach Anspruch 1, wobei das besagte FTY720 oder Derivat in Kombination mit einem oder mehreren Agenzien, die aus Antidepressiva, Angstlösern, Antipsychotika, Antiepileptika, Alzheimer-Medikationen, Parkinson-Medikationen, MAO-Hemmern, Hemmstoffen der Serotonin-Aufnahme, Hemmstoffen der Noradrenalin-Aufnahme, Hemmstoffen der Dopamin-Aufnahme, Dopamin-Agonisten, L-DOPA, Tranquilizern, Sedativa und Lithium ausgewählt sind, verabreicht wird.

14. Verwendung nach Anspruch 1, wobei das besagte FTY720 oder Derivat zur systemischen Verabreichung vorgesehen ist.

15. Verwendung nach Anspruch 1, wobei die besagte Zusammensetzung zur oralen, subkutanen, intraperitonealen, intramuskulären, intraventrikulären, intraparenchymalen, intrathekalen, intrakranialen, bukkalen, mukosalen, nasalen oder rektalen Verabreichung vorgesehen ist.

16. Verwendung nach Anspruch 1, wobei die besagte Zusammensetzung als Nasenspray oder Nasenzäpfchen formuliert ist.

17. Verwendung nach Anspruch 16, wobei die besagte Zusammensetzung zur Verabreichung durch einen Trockenpulver-Inhalator oder einen flüssigkeitsbasierten Inhalator vorgesehen ist.

18. Verwendung nach Anspruch 1, wobei das besagte FTY720 oder ein Derivat davon zur Verabreichung an das Zentralnervensystem des Probanden vorgesehen ist.

19. Verwendung nach einem der Ansprüche 1 bis 18, wobei der Phosphatester von FTY720 aus FTY720P und R-AFD ausgewählt ist.

20. Verwendung nach einem der Ansprüche 1 bis 18, wobei das besagte Derivat von FTY720 aus FTY720P, R-AAL und R-AFD ausgewählt ist.

## Revendications

1. Utilisation de FTY720 ou d'un dérivé de celui-ci choisi parmi le R-AAL, les métabolites ester de phosphate de FTY720, les sels pharmaceutiquement acceptables de FTY720 ou le FTY720 PEGylé dans la fabrication d'une composition pour utilisation dans le traitement d'une maladie neurodégénérative, d'une maladie neurologique ou d'une maladie psychiatrique, à condition que la maladie ne soit pas la sclérose en plaques (SP) ou une maladie démyélinisante telle que le syndrome de Guillain-Barré.

2. Utilisation selon la revendication 1, dans laquelle ladite maladie neurodégénérative, maladie neurologique ou maladie psychiatrique est choisie parmi les troubles neurodégénératifs, les troubles des cellules souches neurales, les troubles des progéniteurs neuronaux, les troubles ischémiques, les troubles affectifs, les troubles neuropsychiatriques, et les troubles de l'apprentissage et de la mémoire.

3. Utilisation selon la revendication 1, dans laquelle ladite maladie neurodégénérative, maladie neurologique ou maladie psychiatrique est choisie parmi la maladie de Parkinson, les troubles parkinsoniens, la maladie de Huntington, la maladie d'Alzheimer, la sclérose latérale amyotrophique, l'ischémie spinale, l'AVC ischémique, les lésions de la moelle épinière, les lésions cérébrales liées à un cancer et les lésions de la moelle épinière liées à un cancer, le syndrome de Shy-Drager, l'ophtalmoplégie supranucléaire progressive, la démence à corps de Lewy, l'AVC, l'infarctus cérébral, la démence par infarctus multiples, la démence gériatrique et la dépression.

4. Utilisation selon la revendication 1 dans laquelle la maladie neurodégénérative est la maladie de Parkinson ou la maladie d'Alzheimer.

5. Utilisation selon la revendication 1 dans laquelle la maladie psychiatrique est la dépression.

6. Utilisation selon la revendication 1, dans laquelle ledit dérivé est le FTY720P.

7. Utilisation selon la revendication 1, dans laquelle ledit FTY720 ou dérivé est destiné à une administration à une concentration de 1 ng/kg/jour à 1 mg/kg/jour, de préférence de 1 µg/kg/jour à 0,1 mg/kg/jour, plus préférablement de 5 µg/kg à environ 0,07 mg/kg.

8. Utilisation selon la revendication 1, dans laquelle ledit FTY720 ou dérivé est destiné à une administration en une quantité de 0,3 mg à 10 mg.

9. Utilisation selon la revendication 1, dans laquelle ledit FTY720 ou dérivé est destiné à une administration à un mammifère.

10. Utilisation selon la revendication 9, dans laquelle ledit FTY720 ou dérivé est destiné à une administration à un humain.

11. Utilisation selon la revendication 10, dans laquelle ledit FTY720 ou dérivé est destiné à une administration à un adulte.

12. Utilisation selon la revendication 1, dans laquelle ledit FTY720 ou dérivé est administré en association avec un ou plusieurs facteurs de croissance.

13. Utilisation selon la revendication 1, dans laquelle ledit FTY720 ou dérivé est administré en association avec un ou plusieurs agents choisis parmi les antidépresseurs, les traitements anti-anxiété, les traitements antipsychotiques, les traitements antiépileptiques, les traitements contre la maladie d'Alzheimer, les traitements contre la maladie de Parkinson, les inhibiteurs de MAO, les inhibiteurs de l'absorption de la sérotonine, les inhibiteurs de l'absorption de la noradrénaline, les inhibiteurs de l'absorption de la dopamine, les agonistes de la dopamine, le L-DOPA, les tranquillisants, les sédatifs et le lithium.

14. Utilisation selon la revendication 1, dans laquelle ledit FTY720 ou dérivé est destiné à une administration systémique.

15. Utilisation selon la revendication 1, dans laquelle ladite composition est destinée à une administration orale, sous-cutanée, intrapéritonéale, intramusculaire, intraventriculaire, intraparenchymale, intrathécale, intracrânienne, buccale, par les muqueuses, nasale ou rectale.

16. Utilisation selon la revendication 1, dans laquelle ladite composition est préparée sous la forme d'un spray nasal ou d'un suppositoire nasal.

17. Utilisation selon la revendication 16, dans laquelle ladite composition est destinée à une administration par un inhalateur de poudre sèche ou un inhalateur à base aqueuse.

18. Utilisation selon la revendication 1, dans laquelle ledit FTY720 ou dérivé de celui-ci est destiné à une administration au système nerveux central du sujet.

19. Utilisation selon l'une quelconque des revendications 1 à 18, dans laquelle l'ester de phosphate de FTY720 est choisi parmi le FTY720P et le R-AFD.

20. Utilisation selon l'une quelconque des revendications 1 à 18, dans laquelle ledit dérivé de FTY720 est choisi parmi le FTY720P, le R-AAL et le R-AFD.
